# EUROPÄISCHE PATENTSCHRIFT

(11) **EP 1 128 798 B1**
(45) Veröffentlichungstag und Bekanntmachung des Hinweises auf die Patenterteilung: **14.07.2004**
(21) Anmeldenummer: 99962141.0
(22) Anmeldetag: 11.11.1999
(51) Int. Cl.: A61K 6/10

(54) **ABFORMMATERIAL**
IMPRESSION MATERIAL
MATERIAU D'EMPREINTE

(30) Priorität: 11.11.1998 DE 19852056
(43) Veröffentlichungstag der Anmeldung: 05.09.2001
(73) Patentinhaber: A. KETTENBACH FABRIK CHEM. ERZEUGNISSE DENTALSPEZIALITÄTEN GmbH & CO. KG, 35713 Eschenburg (DE)
(72) Erfinder: BUBLEWITZ, Alexander, D-35757 Herborn (DE); REBER, Jens-Peter, D-35757 Herborn (DE); HAHN, Rainer, D-72074 Tübingen (DE)
(74) Vertreter: KEIL & SCHAAFHAUSEN
(86) Internationale Anmeldenummer: PCT/EP1999/008663
(87) Internationale Veröffentlichungsnummer: WO 2000/027342

(56) Entgegenhaltungen:
- EP-A- 0 522 341
- EP-A- 0 891 763
- WO-A-97/32536
- WO-A-98/26748
- DE-A- 19 711 314

## Beschreibung

Gegenstand der vorliegenden Erfindung ist ein Abformmaterial zur Verwendung mit einem Abformlöffel.

In WO-A-97/32536 und WO-A-98/52491 wird ein Abformlöffel sowie ein Abformmaterial für diesen Abformlöffel beschrieben.

Das Abformmaterial wird hierbei von den hinteren Enden des Abformlöffels in den zwischen Abformlöffel und Kiefer begrenzten und abgedichteten Formraum eingespeist und unter Unterdruckwirkung durch die Formrinne zum vorderen Ende des Abformlöffels gezogen. Ein Einbringen von Deflektorelementen in Form von Skelettschäumen bewirkt eine zur Zahnachse parallele Komponente der Strömungsgeschwindigkeit des Abformmaterials, wodurch das vollständige Abformen hinterschnittener und tiefere Bereiche begünstigt wird.

Mit derartigen Abformlöffeln erhält man hochpräzise Zahnabdrücke, die gegenüber den nach klassischen Abformverfahren gewonnenen Abdrücken keine eingeschlossenen Flüssigkeiten (Sulcusflüssigkeit, Blut) oder Luftblasen enthalten und die keine mangelhafte Benetzung, Verzüge oder elastische Rückstellung nach der Verformung der plastischen Abformmasse durch Aushärten unter Anpressdruck aufweisen.

Die dünnfließenden Abformmaterialien, die für die klassischen Abformtechniken eingesetzt werden, sind aufgrund ihrer kinetischen und rheologischen Eigenschaften für den Einsatz im neuen Abformlöffel nicht oder nur eingeschränkt verwendbar.

Abformmassen, die üblicherweise für die klassischen Abformverfahren eingesetzt werden bestehen aus einem Mehrkomponentensystem:
α) Di- und Polyalkenylsiloxan
β) Polyhydrogensiloxane
y) Edelmetallkatalysator der 8. Nebengruppe
δ) verstärkende Füllstoffe
ε) nicht verstärkende Füllstoffe.

Weitere Abformmassen sind beispielsweise in EP-A-0 891 763, DE-A-19711314 und WO-A-9826748 offenbart.

Das der Erfindung zugrunde liegende technische Problem ist die Bereitstellung einer Abformmasse, die in den neuartigen Abformlöffeln gemäß WO-A-97/32536 und WO-A-98/52491 eingesetzt werden kann. Ein weiteres Problem ist es, eine Formulierung zu schaffen, die während der Lagerzeit nicht sedimentiert und separiert, deren rheologischen Eigenschaften während der Lagerzeit nahezu unverändert bleiben und die zu einem ausgehärteten Abformmaterial führt, dessen mechanische Eigenschaften den nach DIN 24823 (ISO 4823) geforderten Merkmalen, sowie den geforderten Reißfestigkeiten und Weiterreißfestigkeiten entspricht.

Gelöst wird dieses Problem durch ein Abformmaterial zur Verwendung mit einem Abformlöffel, wobei das Abformmaterial härtbare Komponenten und mindestens einen ersten Füllstoff aufweist und dadurch gekennzeichnet, dass der mindestens erste Füllstoff eine BET Oberfläche von 20 bis 50 m²/g, vorzugsweise 30 bis 40 m²/g aufweist und in einer Menge von 20-30 Gew.% vorliegt, wodurch das Abformmaterial einen Thixotropieindex von ≤1,1 aufweist und eine Viskosität von 1 bis 40 Pas erreicht.

Das erfindungsgemäße Abformmaterial wird in vorteilhafter Weise den Anforderungen, die an eine Verwendung in mit dem neuen Abdrucklöffel gestellt werden gerecht. Insbesondere den Anforderungen, die hinsichtlich Viskosität, Reaktionskinetik, elastischen Eigenschaften und Thixotropie während des Durchfließens durch den Abformlöffel an die Abformmasse gestellt werden. Im einzelnen sind dies:

### a) Viskosität

Die erfindungsgemäßen Abformmaterialien, die im neuen Abformlöffel gemäß WO-A-97/32536 und WO-A-98/52491 zum Einsatz kommen, gewährleisten ein sehr gutes Fließverhalten, das zu einer optimale Abformung führt. Untersuchungen haben gezeigt, dass diese neue Abformmethode um so bessere Abformergebnisse bringt, je niedriger die Mischungsviskosität des eingesetzten Abformmaterials ist.

Im Rahmen der Erfindung werden dünnfließende Formulierungen mit Mischungsviskositäten im Bereich von 1 bis 40 Pas eingesetzt; besonders bevorzugt sind Mischungsviskositäten von 1 bis 10 Pas.

Dünnfließende Abformmaterialien in diesem Viskositätsbereich sind nach dem Stand der Technik bekannt (siehe Tabelle 3).

Die dünnfließensten Zahnabformmaterialien (DIN 24823, ISO 4823) die auf dem Markt erhältlich sind, zeigen eine Mischungsviskosität von 27 bis 1100 Pas. Wie die Mischungsviskositäten der dünnfließensten Abformmaterialien (Permadyne Garant 2:1, Provil L Panasil contact plus und Lastic Xtra superfine) zeigen, liegen diese oberhalb des idealen Viskositätsbereiches von 1 bis 10 Pas. Deshalb zeigen diese Materialien die für die klassischen Abformtechniken wie Korrektur- und Sandwichabformung eingesetzt werden, für diese neue Abformtechnik nur unbefriedigende Abformergebnisse.

### b) Elastische Eigenschaften während des Durchfließens durch den Abformlöffel (Reaktionskinetik)

Bei der neuen Abformtechnik gemäß WO-A-97/32536 und WO-A-98/52491 erfolgt die Befüllung des Abformlöffels von hinten nach vorne. Ein wichtiges Kriterium ist, dass beim Durchfließen des erfindungsgemäßen Abformmaterials durch den Abformlöffel die Mischungsviskosität nahezu konstant bleibt, d.h. es dürfen sich keine oder nur geringe elastischen Eigenschaften durch chemische Vernetzung ausbilden. Auch hier zeigen die für die klassischen Abformtechniken eingesetzten marktgängigen Produkte (Aquasil LV , Xantopren XL, Coltene President light). Als Nachteile dieser Produkte tritt in Erscheinung, dass diese Produkte während des Durchlaufens durch den Abformlöffel gemäß WO-A-97/32536 und WO-A-98/52491 zu schnell vernetzen und somit schon während des Durchfließen des Abformlöffels elastische Eigenschaften ausbilden.

Auch dieser kinetische Effekt führt mit dem neuen Abformlöffel zu unbefriedigenden Abformergebnissen mit den klassischen Abformmaterialien allein.

### c) Thixotropie

Die erfindungsgemäßen Abformmaterialien, die im neuen Abformlöffel gemäß WO-A-97/32536 und WO-A-98/52491 zum Einsatz kommen, gewährleisten eine möglichst niedrige Thixotropie, die zu einer optimalen Abformung nicht unwesentlich beiträgt. Untersuchungen haben gezeigt, dass diese neue Abformmethode um so bessere Abformergebnisse bringt, je niedriger die Thixotropie des eingesetzten Abformmaterials ist. Die nach dem Stand der Technik verwendeten Abformmaterialien für die klassischen Abformverfahren besitzen üblicherweise thixotrope Eigenschaften, (z.B. Panasil contact plus, Thixotropieindex 1,7).

Diese Abformmaterialien zeigen bei der neuen Abformtechnik nur unbefriedigende Abformergebnisse, weil sie beim Durchfließen des Abformlöffels von hinten nach vorne beim Umfließen der Zähne im Schattenbereich zur Fließrichtung Fließfahnen bilden.

Darüber hinaus erfüllt das erfindungsgemäße Abformmaterial für die neue Abformtechnik in Ergänzung zu den oben genannten Eigenschaften weitere Anforderungen, die im folgenden erläutert sind:

### Mechanische Eigenschaften des ausgehärteten Abformmaterials

Die erfindungsgemäßen Abformmaterialien für die neue Abformtechnik gemäß WO-A-97/32536 und WO-A-98/52491 erfüllen die in DIN 24823 bzw. ISO 4823 nach Typ 3 gestellten Anforderungen hinsichtlich Verformung unter Druck im Bereich von 2 bis 20% und Rückstellung nach der Verformung im Bereich von 96,5 bis 100 %, lineare Maßänderung (Polymerisationsschrumpf) im Bereich von 0 bis 1,5% und eine Wiedergabegenauigkeit von 0,02 mm.

Die Shore Härte (DIN 53505, ISO 868) des ausgehärteten erfindungsgemäßen Abformmaterials liegt zwischen Shore A 30 bis 50, bevorzugt 35 bis 45, was für das neue Abformverfahren gemäß WO-A-97/32536 und WO-A-98/52491 günstig ist.

Einige Abformmaterialien nach dem Stand der Technik, die für die klassischen Abformtechniken eingesetzt werden weisen Shore A Härten >50 auf. (Aquasil LV: Shore A 58, Coltene, President light body: Shore A 59). Hohe Shore A Härten erschweren die Mundentnahme des ausgehärteten Abformmaterials.

Für das neue Abformverfahren gemäß WO-A-97/32536 und WO-A-98/52491 werden die erfindungsgemäßen Abformmaterialien eingesetzt, deren Reißfestigkeit und Weiterreißfestigkeiten (nach DIN /EN 53504 u. DIN 53515) in ausgehärteten Zustand Mindestwerte von 150 N/cm² bis 250 N/cm² bzw. 0,5 - 2,5 N/mm erzielen.

Bei der neuen Abformtechnik nach WO-A-97/32536 und WO-A-98/52491 werden die Zähne von fließfähigen Materialien detailgenau abgebildet. Zahnzwischenräume und untersichgehende Stellen werden vollständig mit Abformmaterial gefüllt. Nach Aushärten des Abformmaterials muss die Reißfestigkeit und Weiterreißfestigkeit eine zerstörungsfreie Mundentnahme und Entnahme aus dem Abformlöffel ermöglichen. Andererseits dürfen Reiß- und Weiterreißfestigkeit nicht zu hohe Werte aufweisen, um den Zahnhalteapparat des Patienten bei der Mundentnahme des ausgehärteten Abformmaterials nicht über Gebühr zu belasten.

### Die Abformmaterialien für die neue Abformtechnik

Wie bereits oben beschrieben werden für die neue Abformtechnik gemäß WO-A-97/32536 und WO-A-98/52491 besonders hohe Anforderungen an die rheologischen Eigenschaften gestellt. Überraschenderweise befriedigt das erfindungsgemäße Abformmaterial diese Anforderungen,

Im folgenden werden bevorzugte Ausführungsformen des erfindungsgemäßen Abformmaterials beschrieben.

Ein geeignetes Abformmaterial für die neue Abformtechnik weist eine sehr niedrige Mischungsviskosität (zwischen 1 und 10 Pas) und keine oder nur sehr geringe Thixotropieeigenschaften auf (Thixotropieindex ≤ 1,1).

Eine Mischungsviskosität zwischen 1 und 10 Pas bedeutet für das Mehrkomponentenmaterial, in der Regel ein Zweikomponentenmaterial, dass die Viskositäten der Einzelkomponenten kleiner oder gleich 1 bis 10 Pas und die Thixotropie kleiner oder gleich 1,1 sind.

Im Gegensatz zu den nicht oder wenig thixotropen Einzelkomponenten von Abformmaterialien im oben genannten Viskositätsbereich von 1 bis 10 Pas nach dem Stand der Technik neigen die erfindungsgemäßen Abformmaterialien nicht zur Sedimentation der Füllstoffe und zur Separation der Polymere bzw. Weichmacher während der Lagerzeit.

Die Haltbarkeitsdauer von dentalen Abformmaterialien in Primärgebinden sollte dabei mindestens 18 Monate bis 36 Monate betragen. Über diesen Zeitraum hinweg dürfen die genannten Separations- und Sedimentationsphänomene nicht auftreten, da sonst die Qualitätsmerkmale des Abformmaterials leiden. Ferner dürfen die eingestellten rheologischen Eigenschaften während der Lagerzeit nur innerhalb geringer Toleranzen abweichen, um die geforderten Fließeigenschaften zu gewährleisten.

Vor allem das in der Praxis häufig auftretende Phänomen des Nachversteifens, d.h. Ansteigen der Viskosität und Thixotropie im Laufe der Lagerzeit, sollte weitestgehend vermieden werden.

Eine gewisse leicht abbaubare Thixotropie der Einzelkomponenten des erfindungsgemäßen Abformaterials ist akzeptabel und bietet sogar Vorteile bei der Lagerstabilität hinsichtlich Sedimentation und Separation, da bei thixotropen Einzelkomponenten die Füllstoffpartikel in der Schwebe gehalten werden.

Voraussetzung ist, dass sich die Thixotropie der Einzelkomponenten nicht auf die Thixotropie der gemischten Abformmasse auswirkt, da sonst die Fließfähigkeit des Abformmaterials im neuen Abformlöffel beeinträchtigt wird.

Das erfindungsgemäße Abformmaterial erfüllt die folgenden Bedingungen:

Die Mischungsviskosität liegt zwischen 1 bis 350 Pas, das Abformmaterial weist keine oder nahezu keine Thixotropieeigenschaften auf, die rheologischen Eigenschaften bleiben während der Lagerzeit nahezu unverändert und es tritt während der Lagerzeit keine Sedimentation oder Separation auf.

Weiterhin genügen die mechanischen Eigenschaften des ausgehärteten Abformmaterials hinsichtlich Reißfestigkeit, Weiterreißfestigkeit, Shore A Härte und den Anforderungen nach DIN EN 24823 (ISO 4823) den o.g. Anforderungen.

In einer bevorzugten Ausführungsform beträgt die Viskosität der Mischung 1 bis 40 Pas, insbesondere bevorzugt 1 bis 10 Pas. Um die geforderten Mischungsviskositäten von 1 bis 350 Pas, insbesondere 1 bis 40 Pas, vorzugsweise 1 bis 10 Pas zu realisieren, bedeutet dies für die Einzelkomponenten eines Zweikomponenten Abformmaterials, dass diese Einzelviskosität zwischen den genannten Grenzen liegt. Es sind auch Einzelviskositäten denkbar, bei denen die eine Komponente sehr hohe und die andere Komponente sehr niedrige Viskositäten besitzt. Erfindungsgemäß muss die Mischung dann eine Viskosität von 1 bis 40 Pas, vorzugsweise 1 bis 10 Pas aufweisen.

Erfindungsgemäß kann ein dünnfließendes, nicht thixotropes Abformmaterial mit einer Mischungsviskosität mit den genannten Grenzen und einem Thixotropieindex von 1,0 bis 1,1, dessen rheologische Eigenschaften während der Lagerzeit unverändert bleiben, das während der Lagerzeit nicht sedimentiert und separiert und dessen mechanische Eigenschaften nach Aushärtung den nach ISO 4823 geforderten Merkmalen, sowie den geforderten Reißfestigkeiten und Weiterreißfestigkeiten entspricht, hergestellt werden.

Dies gelingt vorzugsweise durch Einsatz eines ersten anorganischen Füllstoffs mit einer BET-Oberfläche zwischen 20 und 50 m²/g, der hydrophobiert sein kann, in dem erfindungsgemäßen Abformmaterial.

Besonders bevorzugt ist eine Kombination dieses anorganischen Füllstoffs mit einem verstärkenden anorganischen Füllstoff mit einer BET-Oberfläche die größer als diejenige des ersten Füllstoffes ist und die vorzugsweise zwischen 50 bis 700 m²/g, insbesondere 110 bis 170 m²/g liegt.

Gegenstand der vorliegenden Erfindung ist auch eine Füllstoffkombination aus einem ersten und einem zweiten Füllstoff, wobei der erste anorganische Füllstoff eine BET-Oberfläche von 20 bis 50 m²/g und der zweite Füllstoff ein anorganischer verstärkender Füllstoff ist, der eine größere BET-Oberfläche besitzt als die Oberfläche des gewählten ersten Füllstoffes. Vorzugsweise hat der zweite Füllstoff eine BET-Oberfläche von 50 bis 700 m²/g, insbesondere 110 bis 170 m²/g.

Der erfindungsgemäße anorganische Füllstoff weist insbesondere folgende Merkmale auf:

Die Dichte beträgt 2,0 bis 2,2 g/cm³, die BET-Oberfläche beträgt zwischen 20 bis 50 m²/g, insbesondere zwischen 30 und 40 m²/g, z. B. ungefähr 35 m²/g. Die BET-Oberfläche besitzt aufgrund der produktionstechnischen Gegebenheiten eine Schwankungsbreite, deren Mittelwert hier zugrundegelegt wird. Typischerweise beträgt die Schwankungsbreite ± 5 bis 15 m²/g. Die Dibutylphalat-Adsorption nach DIN 53601 beträgt zwischen 140 und 180 g/100g. Die Öladsorption nach DIN ISO 7875 beträgt zwischen 35 bis 60 g/100. Die mittlere Korngröße liegt zwischen 0,5 und 20 µm.

Bei dem erfindungsgemäßen anorganischen Füllstoff handelt es sich vorzugsweise um eine naßgefällte Kieselsäure oder um eine natürlich vorkommende Kieselsäure. Diese können im Vergleich zu pyrogener Kieselsäure einen relativ hohen Wassergehalt von 2 bis 8 % aufweisen.

Die erfindungsgemäßen anorganischen Füllstoffe bestehen zu 80 bis 100% aus Siliciumdioxid. Daneben können auch andere Metalloxide wie Aluminiumoxid, Calciumoxid, Natriumoxid, Kaliumoxid, Eisenoxid und Titandioxid in Prozentbereich von 0 bis 20% enthalten sein. Besonders bevorzugt als zusätzliche Metalloxide neben Siliciumdioxid sind Calciumoxid, Natriumoxid, Kaliumoxid und Aluminiumoxid.

Die erfindungsgemäß in Kombination mit dem erfindungsgemäßen anorganischen verstärkenden Füllstoff mit einer BET-Oberfläche von 20 bis 50 m²/g einzusetzende hydrophobierte Kieselsäure hat insbesondere folgende Merkmale:

Die Dichte beträgt zwischen 2,0 und 2,2 g/cm³, die BET-Oberfläche beträgt zwischen 110 und 170 m²/g insbesondere zwischen 130 und 150 m²/g.

Die Primärteilchengröße liegt zwischen 5 bis 30 nm.

Die hydrophobierte hochdisperse Kieselsäure wird vorzugsweise durch eine Flammenhydrolyse von Siliciumtetrachlorid hergestellt.

Es handelt sich um eine hydrophobierte hochdisperse Kieselsäure, die als synthetisches röntgenamorphes Siliciumdioxid vorliegt.

Die Oberflächenbehandlung der hydrophobierten hochdispersen Kieselsäure erfolgt vorzugsweise mit Trimethylsilylgruppen. Der Hydrophobierungsgrad ist durch den Kohlenstoffgehalt von 1 bis 6 % und bevorzugt zwischen 2 und 3 % charakterisiert.

Vorzugsweise wird diese hydrophobierte Kieselsäure bei ihrer Herstellung einem zusätzlichen Veredelungsschritt unterzogen. Es wird eine starke mechanische Behandlung über Kollergang oder Kugelmühle durchgeführt.

Das erfindungsgemäße Abformmaterial auf Basis additionsvernetzender Silicone für die Abformtechnik mit dem neuen Abformlöffel besteht vorzugsweise aus folgenden Inhaltsstoffen:
α) Di- und Polyalkenyl―Siloxane
β) Polyhydrogensiloxane
y) Edelmetallkatalysator der 8. Nebengruppe
δ) gegebenenfalls verstärkender Füllstoff in Form von hydrophobierter hochdisperser Kieselsäure mit einer BET-Oberfläche von 110 bis 170 m²/g.
ε) anorganischer Füllstoff mit einer BET-Oberfläche von 20 bis 50 m²/g, der hydrophobiert sein kann
sowie gegebenenfalls Hilfstoffen wie Weichmacher, oberflächenaktive Tenside, Farbstoffe und Polyether-Siloxan-Copolymere, die gegebenenfalls Vinyl- oder SiH-Gruppen enthalten.

Die Inhaltsstoffe werden in der Regel auf zwei getrennte Komponenten aufgeteilt:
z.B. A-Komponente α) + γ) + ε) + δ)
B-Komponente β) + ε) + δ) und eventuell α).

Besonders bevorzugt für das erfindungsgemäße Abformmaterial ist folgende Zusammensetzung von Inhaltsstoffen:
α)α,ω-vinylendgestoppte Polydimethylsiloxane in einem Prozentbereich von 40 bis 80%, insbesondere 50 bis 70%.
β) Polyhydrogenpolydimethylsiloxane mit mindestens 2 SiH-Gruppen bzw. einem SiH-Gehalt von 0,1 bis 15 mmol/g in einem Prozentbereich von 2 bis 40%, insbesondere von 10 bis 30%.
γ) Katalysatoren für die Hydrosilylierung, Salze, komplexe und kolloidal vorliegende Formen der Übergangsmetalle der 8. Nebengruppe, vorzugsweise die Metalle Platin, Palladium und Rhodium, insbesondere Platinkomplexe, die z.B. aus Hexachloroplatinsäure bzw. aus Platinsalzen hergestellt werden, (Karstedt Katalysatoren) in einem Prozentbereich von 0,0001 bis 0,1%, insbesondere 0,0005 bis 0,1% bezogen auf reines Metall.
δ) Gegebenenfalls verstärkender Füllstoff in Form von hydrophobierter hochdisperser Kieselsäure mit einer BET-Oberfläche von 130 bis 150 m²/g in einem Gewichtsbereich von 0 bis 30%.
ε) Anorganischer Füllstoff mit einer BET-Oberfläche von 30 bis 40 m²/g, der hydrophobiert sein kann in einem Prozentbereich von 10 bis 50%, insbesondere 20 bis 30%.

Es ist an sich bekannt, dass die Anwesenheit von Säuren, Basen oder Wasser bereits in geringen Konzentrationen den Platin-Katalysator irreversibel schädigen. Dies zeigt sich im Verlaufe längerer Stabilitätseinlagerung unter Raumtemperaturbedingungen nach ca. 6 Monaten oder bei Temperatur Stresstest z.B. 60°C bereits nach 1 bis 2 Wochen dahingehend, dass die Vernetzungsreaktion des 2-Komponentenmischers stark verzögert ist oder völlig zum Erliegen kommt.

Dieser Effekt ist für eine dentale Abformmasse nicht tolerierbar. Säuren, Wasser und vor allen Dingen Basen initiieren die Wasserstoffabspaltung von Hydrogenpolysiloxanen.

Dies führt zum einen zum Aufblähen des Primärgebindes aufgrund der Wasserstoffgasentwicklung und zum anderen führt dies bei der Additionsreaktion des 2-Komponentenmaterials aufgrund fehlender SiH-Funktionalitäten zu einer verlangsamten Vernetzungsreaktionen und zu einer Schwächung des ausgehärteten Silicongummis.

Überraschenderweise lassen sich erfindungsgemäß auch anorganische Füllstoffe mit einer BET-Oberfläche von 20 bis 50 m²/g einsetzen, die einen hohen Wassergehalt von bis zu 10% und/oder einen hohen pH-Wert von bis zu 11 aufweisen.

Nach entsprechender Trocknung solcher Füllstoffe, sind Formulierungen von additionsvernetzenden Siliconen mit diesen Füllstoffen stabil.

Die den Platinkatalysator enthaltende Komponente A eines Zweikomponenten-Abformmaterials zeigt innerhalb der Lagerzeit von bis zu 36 Monaten keine Schädigung der Abbindekinetik. Bei der Komponente B, die Polyhydrogensiloxane enthält, ist während dieser Lagerzeit keine Wasserstoffentwicklung festzustellen.

Das erfindungsgemäße Abformmaterial (siehe Beispiel 27,33) zeichnet sich durch eine sehr gute Fließfähigkeit aus. Die Mischungsviskosität ist je nach Kettenlänge der eingesetzten Siliconpolymeren zwischen 1 und 40 Pas einstellbar, besonders gute Abformergebnisse lassen sich mit Mischungsviskositäten zwischen 1 und 10 Pas erzielen.

Die Abformmasse besitzt in gemischten Zustand keine relevanten thixotropen Eigenschaften. Mischungsviskosität und die nicht relevanten thixotropen Eigenschaften bleiben während der Lagerzeit nahezu unverändert erhalten.

Durch den Einsatz des erfindungsgemäßen anorganischen Füllstoffs wird die Sedimentation des Füllstoffs und die Separation der Siliconpolymere während der Lagerzeit von bis zu 36 Monaten bei Raumtemperatur verhindert.

Die Abbindecharakteristik des Abformmaterials kann bei dem erfindungsgemäßen Abformmaterial in an sich bekannter Weise in weiten Bereichen eingestellt werden, z.B. durch Einsatz und Auswahl von Inhibitoren.

Die Verarbeitungszeit, d.h. die Zeit die das Abformmaterial zum Durchfließen des neuen Abformlöffels benötigt, liegt zwischen 20 und 120 s, bevorzugt zwischen 25 und 60 s.

Das Abformmaterial härtet nach vollständiger Befüllung des neuen Abformlöffels im Patientenmund innerhalb von 2,0 bis 10,0 min aus. Besonders bevorzugt sind Abbindezeiten zwischen 2,0 und 4,0 min., da hierdurch der Zeitbedarf für den behandelnden Zahnarzt und die Belastung für den Patienten am geringsten sind.

Gegenüber den Abformmaterialien nach dem Stand der Technik zeichnet sich das erfindungsgemäße Abformmaterial dadurch aus, dass die Mischungsviskosität des Abformmaterials beim Durchfließen des Abformlöffels nahezu konstant bleibt, d.h. die Reaktionskinetik ist so eingestellt, dass sich in der Zeit des Durchfließens keine oder nur sehr geringe elastische Eigenschaften durch chemische Vernetzung ausbilden. Nach Beendigung der Durchflussphase härtet das erfindungsgemäße Abformmaterial vorzugsweise sehr schnell aus (snap-Effekt). Die mechanischen Eigenschaften des erfindungsgemäßen Abformmaterials erfüllen die Anforderungen nach DIN EN 24823, (ISO 4823). Die Rückstellung nach der Verformung liegt zwischen 96,5 und 100,0%, insbesondere zwischen 99,0 und 100%. Die Verformung unter Druck liegt zwischen 2,0 bis 20,0%, insbesondere zwischen 5,0 und 15,0.

Die Shore A Härte des erfindungsgemäßen Abformmaterials liegt zwischen 30 und 50, insbesondere zwischen 35 und 45. Die Reiß- und Weiterreißfestigkeit des erfindungsgemäßen Abformmaterials liegen zwischen 150 bis 250 N/cm² bzw. 0,5 bis 2,5 N/mm. Diese mechanischen Eigenschaften des erfindungsgemäßen Abformmaterials gewährleisten nach Aushärten des Abformmaterials eine zerstörungsfreie Entnahme des Abformlöffels aus dem Patientenmund und eine Entnahme des Abdrucks aus dem Abformlöffel. Andererseits sind die mechanischen Eigenschaften so ausgebildet, dass eine Schädigung des Zahnhalteapparates des Patienten bei der Mundentnahme nicht auftreten kann.

Im folgenden werden die einzelnen Komponenten der erfindungsgemäßen Abformmaterialien untersucht, zur Erläuterung der Bedeutung der Abmischungen und deren Optimierung.

Vergleichsbeispiele:
a) Formulierungen, die aufgebaut sind aus Vinylsiloxan, SiH-Siloxan, Platin-Katalysator, die keine Füllstoffe enthalten, bei deren Formulierungen können die Kettenlänge der eingesetzten Siliconpolymeren in den Einzelkomponenten derart variieren, so dass die Mischungsviskosität zwischen den oben genannten Grenzen, insbesondere bevorzugt bei 1 bis 10 Pas liegt. Die Mischung derartiger Formulierungen zeigt sehr gute Fließeigenschaften. Es liegt keine Thixotropie vor.
   Jedoch sind die mechanischen Eigenschaften des ausgehärteten Abformmaterials unabhängig vom Vernetzungsgrad völlig unzureichend (siehe Vergleichsbeispiel 3).
   Bei allen folgenden Beispielen wurde die maximal mögliche Konzentration an verstärkenden bzw. nicht verstärkenden Füllstoffen zugesetzt, die gerade noch mit dem oberen Limit der Mischungsviskosität einstellbar ist.
   In allen Fällen wurden mit Trimethylsil'ylgruppen hydrophobile Kieselsäuren und Füllstoffe eingesetzt um eine maximale Kompatibilität zur Siliconmatrix zu erhalten.
b) Formulierungen, die aufgebaut sind aus Vinylsiloxan, SiH-Siloxan, Pt-Katalysator und verstärkenden Füllstoffen.
   Hier zeigt sich folgendes Bild:
   Bei niedrigen und hohen Konzentrationen der hochdispersen Kieselsäure (verstärkender Füllstoff) werden wie unter a) beschrieben zwar die gewünschten Mischungsviskositäten erreicht, allerdings sind auch hier die mechanischen Eigenschaften zwar schon wesentlich verbessert gegenüber a) jedoch noch nicht zufriedenstellend erfüllt (siehe Vergleichsbeispiel 6 und 24).
c) Formulierungen, die aufgebaut sind aus Vinylsiloxan, SiH Siloxan, Pt-Katalysator und nicht verstärkende Füllstoffen.
   Die gewünschten Mischungsviskositäten sind selbst bei hohen Konzentrationen an nicht verstärkenden Füllstoffen erreichbar, allerdings sind die mechanischen Eigenschaften nicht für eine Anwendung in der neuen Abformtechnik geeignet. Darüber hinaus sedimentieren die eingesetzten nicht verstärkenden Füllstoffe im Verlauf des Lagertests (siehe Vergleichsbeispiele 7,8,9).
d) Formulierungen, die aufgebaut sind aus Vinylsiloxan, SiH-Siloxan, Pt-Katalysator und Kombinationen aus einem verstärkenden und einem nicht verstärkenden Füllstoff.
d1) In einem Grenzfall der möglichen Kombination wird eine hohe Konzentration eines verstärkenden Füllstoffs und eine niedrige Konzentration eines nicht verstärkenden Füllstoffs eingesetzt.
   Die gewünschte Mischungsviskosität lässt sich einstellen allerdings sind die mechanischen Eigenschaften des ausgehärteten Abformmaterials ebenfalls unzureichend. Darüber hinaus tritt hier schon die Tendenz zur Sedimentation der nicht verstärkenden Füllstoffe im Lagertest auf (siehe Vergleichsbeispiel 10,11 und 12).
d2) Im zweiten Grenzfall wird eine niedrige Konzentration eines verstärkenden Füllstoffs mit einer hohen Konzentration eines nicht verstärkenden Füllstoffs kombiniert.
   Die gewünschte Mischungsviskosität lässt sich einstellen, allerdings sind die mechanischen Eigenschaften des ausgehärteten Abformmaterials nicht für eine Verwendung in der neuen Abformtechnik geeignet. Zusätzlich tritt eine starke nicht akzeptable Sedimentationsneigung im Lagertest auf (siehe Vergleichbeispiel 16,17,18, 19, 20 und 21).
d3) Bei Einsatz von mittleren Konzentration von nicht verstärkenden und verstärkenden Füllstoffen lässt sich die gewünschte Mischungsviskosität ebenfalls einstellen.
   Bei Einlagerung erhöht sich die Mischungsviskosität des Abformmaterials, so dass die Fliesseigenschaften und damit das Abformergebnis über die Lagerzeit nicht gewährleistet sind.
   Es ergeben sich am ausgehärteten Abformmaterial mittelmäßige aber noch nicht ausreichende mechanische Eigenschaften.
   Im Lagertest tritt eine mittlere Sedimentationsneigung (siehe Vergleichsbeispiele 13,14 und 15) auf.

Wie die oben genannten Ausführungen zeigen, erfüllt keine der Formulierungen, die sich im Mischungs- Viskositätsbereich für das Abformmaterial gemäß der Erfindung, vorzugsweise zwischen 1 bis 10 Pas, bewegen die übergeordneten Anforderungen hinsichtlich der mechanischen Eigenschaften des ausgehärteten Abformmaterial und der Lagerstabilität hinsichtlich Nachversteifen, Separation und Sedimentation.

Die an sich durch die Konzentrationserhöhung von verstärkenden und nicht verstärkenden Füllstoffen hervorgerufenen positiven Eigenschaften, gehen also mit rheologischen Nachteilen einher; d.h. durch Erhöhung der Konzentration der verstärkenden Füllstoffe erhöht sich die Viskosität der Einzelkomponente drastisch. Außerdem steigt die Tendenz zur Nachversteifung während der Lagerzeit. Bei nicht verstärkenden Füllstoffen tritt eine Sedimentation der Füllstoffe während der Lagerzeit auf und eine Separation der Polymere. Zusätzlich wird die Reißund Weiterreißfestigkeit beeinträchtigt.

Es wird insbesondere auf die nach den Beispielen folgenden Tabellen verwiesen, die die Ergebnisse der Beispiele zusammenfassend wiedergeben.

Die Erfindung wird anhand der folgenden Beispiele näher beschrieben:

### Beispiel 1 (Vergleichsbeispiel)

Komponente A eines Zahnabformmaterials, das keine Füllstoffe enthält.

In einem Vakuummischer werden 99 Teile eines vinylendgestoppten Polydimethylsiloxans mit einer Viskosität von 7,0 Pas bei 20 °C mit einem Teil eines Platinkatalysators vom Karstedt - Typ mit einem Gehalt an reinem Platin von 1 % für 15 Minuten unter Vakuum homogen gemischt.

Man erhält eine dünnfließende Paste mit einer Viskosität von 6,4 Pas, die keine Thixotropie (Thixotropieindex 1,0) aufweist.

Die Paste zeigt während der Lagerzeit keine Veränderung der rheologischen Eigenschaften und weist naturgemäß keine Sedimentation oder Separation auf.

### Beispiel 2 (Vergleichsbeispiel)

Komponente B eines Zahnabformmaterial, das keine Füllstoffe enthält.

In einem Vakuummischer werden 82 Teile eines vinylendgestoppten Polydimethylsiloxans mit einer Viskosität von 7,0 Pas bei 20 °C mit 18 Teilen eines Polymethylhydrogensiloxans mit einer Viskosität von 0,2 Pas und einem SiH-Gehalt von 1,8 mmol/g und 0,02 Teilen Divinyltetramethyldisiloxan für 15 Minuten unter Vakuum homogen gemischt.

Man erhält eine dünnfließende Paste mit einer Viskosität von 4,6 Pas die keine Thixotropie aufweist (Thixotropieindex 1,0).

Die Paste weist während der Lagerzeit keine Veränderung der rheologischen Eigenschaften und naturgemäß keine Sedimentation oder Separation auf.

### Beispiel 3 (Vergleichsbeispiel)

Mischung der Komponente A aus Beispiel 1 und der Komponente B aus Beispiel 2.

50 Teile der Komponente A aus Beispiel 1 und 50 Teile der Komponente B aus Beispiel 2 werden aus einer Doppelkammerkartusche über einen statischen Mischer ausgedrückt und homogen gemischt. Man erhält eine dünnfließende Paste mit einer Mischungsviskosität von 5,0 Pas, die keine Thixotropie aufweist (Thixotropieindex 1,0). Die Paste besitz bei 35°C eine Verarbeitungszeit von 60 s und ist bei 35 °C nach 240 s vollständig ausgehärtet.

Man erhält nach vollständiger Aushärtung transparente, weiche, wenig elastische und brüchige Formkörper. Die Rückstellung nach der Verformung nach ISO 4823 des ausgehärteten Abformmaterials liegt bei 99,80 %. Die Verformung unter Druck nach ISO 4823 liegt bei 11,0 %. Die Shore A Härte liegt bei 18.
Die Reiß- und Weiterreißfestigkeit ist nicht meßbar, da die Prüfkörper bei Entnahme aus der Prüfkörperform bzw. beim Einspannen in das Prüfgerät brechen bzw. einreißen.

Diese mechanischen Eigenschaften sind unzureichend für ein Zahnabformmaterial.

Bei Mundentnahme und Entnahme des Abdrucks aus dem Abformlöffel bricht bzw. reißt das Material ein.
Ein solches Material ist für die Abformtechnik mit dem neuen Abformlöffel ungeeignet. Das rheologische und kinetische Verhalten des Abformmaterials, sowie dessen Verhalten im Lagertest hinsichtlich Sedimentation, Separation und Nachversteifung ist zwar gut, jedoch ist die mechanische Festigkeit der erhaltenen Vulkanisate vollkommen unzureichend.

### Beispiel 4 (Vergleichsbeispiel)

Komponente A eines Zahnabformmaterials, das als Füllstoff nur verstärkenden Füllstoff enthält.

In einem Vakuummischer werden 79 Teile eines vinylendgestoppten Polydimethylsiloxans mit einer Viskosität von 1,0 Pas bei 20°C mit 1 Teil eines Platinkatalysators vom Karstedt - Typ mit einem Gehalt an reinem Platin von 1% mit 21 Teilen einer hydrophobierten (mit Trimethylsilylgruppen oberflächenbehandelten), hochdispersen Kieselsäure mit einer BET - Oberfläche von 140 m²/g für 60 Minuten unter Vakuum homogen gemischt.

Man erhält eine dünnfließende Paste mit einer Viskosität von 10,0 Pas, die nahezu keine Thixotropie aufweist (Thixotropieindex 1,0). Die Paste zeigt während der Lagerzeit von einer Woche 60 °C eine leichte Erhöhung der Viskosität auf 11,2 mPas und eine gleichbleibende Thixotropie auf (Thixotropieindex 1,0). Die Paste weist während der Lagerzeit keine Sedimentation oder Separation auf.

### Beispiel 5 (Vergleichsbeispiel)

Komponente B eines Zahnabformmaterials, das als Füllstoff nur verstärkenden Füllstoff enthält.
In einem Vakuummischer werden 58 Teile eines vinylendgestoppten Polydimethylsiloxans mit einer Viskosität von 0,2 Pas bei 20 °C mit 27 Teilen eines verzweigten Polymethylhydrogensiloxans mit einer Viskosität von 0,2 Pas und einem SiH-Gehalt von 4,3 mmol/g, mit 19 Teilen einer hydrophobierten (mit Trimethylsilylgruppen oberflächenbehandelten), hochdispersen Kieselsäure mit einer BET - Oberfläche von 140 m²/g und 0,01 Teilen eines Lebensmittelfarbstoffs für 60 Minuten unter Vakuum homogen gemischt.

Man erhält eine dünnfließende Paste mit einer Viskosität von 9,8 Pas die geringe Thixotropie aufweist (Thixotropieindex 1,1). Die Paste zeigt während der Lagerzeit von einer Woche 60°C eine deutliche Erhöhung der Viskosität auf 15,0 Pas und einen Thixotropieindex von 1,1 auf. Die Paste weist während der Lagerzeit keine Sedimentation oder Separation auf.

### Beispiel 6 (Vergleichsbeispiel)

Mischung der Komponente A aus Beispiel 4 und der Komponente B aus Beispiel 5.

50 Teile der Komponente A aus Beispiel 4 und 50 Teile der Komponente B aus Beispiel 5 werden aus einer Doppelkammerkartusche über einen statischen Mischer ausgedrückt und homogen gemischt. Man erhält eine dünnfließende Paste mit einer Mischungsviskosität von 9,0 Pas, die keine Thixotropie aufweist (Thixotropieindex 1,0). Die Paste besitzt bei 35 °C eine Verarbeitungszeit von 30 s und ist bei 35 °C nach 240 s vollständig ausgehärtet.
Man erhält nach vollständiger Aushärtung transparente, weiche, elastische Formkörper. Die Rückstellung nach der Verformung liegt bei 99,60 % (ISO 4823). Die Verformung unter Druck nach ISO 4823 liegt bei 5,45%. Die Shore A-Härte liegt bei 37. Die Reiß- und Weiterreißfestigkeit liegt bei 103 N/cm² bzw. 0,41 N/mm.

Diese mechanischen Eigenschaften sind für ein Zahnabformmaterial für den neuen Abformlöffel nicht ausreichend. Bei Mundentnahme und Entnahme des Abdrucks aus dem Abformlöffel besteht die Gefahr, dass das Material bricht bzw. einreißt.
Das rheologische Verhalten des Abformmaterials nach dem Lagertest zeigt eineMischungsviskosität von 8,3 Pas und einen Thixotropieindex 1,0 während der Lagerzeit (1 Woche 60°C). Die Abbindecharakteristik des Abformmaterials verändert sich während der Lagerzeit nahezu nicht. Dieses Abformmaterial ist für die Abformtechnik mit dem neuen Abformlöffel nicht geeignet.
Das rheologische und kinetische Verhalten des Abformmaterials, sowie dessen Verhalten im Lagertest hinsichtlich Sedimentation, Separation und Nachversteifung ist zwar gut, jedoch ist die mechanische Festigkeit der erhaltenen Vulkanisate vollkommen unzureichend.

### Beispiel 7 (Vergleichsbeispiel)

Komponente A eines Zahnabformmaterials, das nur nicht verstärkenden Füllstoff enthält.

In einem Vakuummischer werden 40 Teile eines vinylendgestoppten Polydimethylsiloxans mit einer Viskosität von 1000 mPas bei 20°C mit 1 Teil eines Platin - Katalysators vom Karstedt - Typ mit einem Gehalt an reinem Platin von 1% mit 59 Teilen eines mit Trimethylsilylgruppen oberflächenbehandelten Siliciumdioxid Füllstoffs (Quarz) mit einer BET - Oberfläche von < 1m² /g und einer mittleren Korngröße von 10 µm für 60 Minuten unter Vakuum homogen gemischt. Man erhält eine dünnfließende Paste mit einer Viskosität von 8,7 Pas, die geringe Thixotropie aufweist (Thixotropieindex 1,1). Die Paste zeigt während der Lagerzeit von 1 Woche 60°C eine deutliche Sedimentation des Füllstoffs und Separation des Siliconpolymers. Weiterhin zeigt sich im Lagertest eine leichte Erhöhung der Viskosität auf 11,8 Pas und der Thixotropie (Thixotropieindex 1,1).

### Beispiel 8 (Vergleichsbeispiel)

Komponente B eines Zahnabformmaterials, das als Füllstoff nur nicht verstärkenden Füllstoff enthält. In einem Vakuummischer werden 29 Teile eines vinylendgestoppten Polydimethylsiloxans mit einer Viskosität von 1000 mPas bei 20°C mit 6 Teilen eines Polymethylhydrogensiloxans mit einer Viskosität von 200 mPas bei 20°C und einem SiH-Gehalt von 1,8 mmol/g und 65 Teilen eines mit Trimethylsilylgruppen oberflächenbehandelten Siliciumdioxid - Füllstoffs (Quarz) mit einer Oberfläche von < 1m²/g und einer mittleren Korngröße von 10 µm und 0,01 Teile eines Lebensmittelfarbstoffs für 60 Minuten unter Vakuum homogen gemischt.

Man erhält eine dünnfließende Paste mit einer Viskosität von 8,4 Pas, die geringe Thixotropie aufweist (Thixotropieindex 1,2) Die Paste zeigt während der Lagerzeit von 1 Woche 60 °C eine deutliche Sedimentation des Füllstoffs und eine Separation des Siliconpolymers. Weiterhin zeigt sich im Lagertest nahezu keine Veränderung der Viskosität von 8,6 Pas und der Thixotropie ( Thixotropieindex 1,2).

### Beispiel 9 (Vergleichsbeispiel)

Mischung der Komponente A aus Beispiel 7 und der Komponente B aus Beispiel 8.

50 Teile der Komponente A aus Beispiel 7 und 50 Teile der Komponente B aus Beispiel 8 werden aus einer Doppelkammerkartusche über einen statischen Mischer ausgedrückt und homogen gemischt. Man erhält eine dünnfließende Paste mit einer Mischungsviskosität von 10,0 Pas, die eine geringe Thixotropie aufweist (Thixotropieindex 1,1). Die Paste besitzt bei 35°C eine Verarbeitungszeit von 60s und ist bei 35°C nach 230 Sekunden vollständig aushärtet.

Man erhält nach vollständiger Aushärtung relativ harte, wenig elastische Formkörper. Die Rückstellung nach der Verformung liegt nach ISO 4823 bei 99,4 %. Die Verformung unter Druck liegt nach ISO 4823 bei 1,9 %. Die Shore A-Härte beträgt 73.Die Reiß- und Weiterreißfestigkeit liegt bei 308 N/cm² bzw. 0,77 N/mm.
Die Reißfestigkeiten sind für eine Zahnabformung mit dem neuen Abformlöffel an der oberen Grenze, die Shore A-Härte ist zu hoch, so dass bei Mundentnahme die Gefahr der Schädigung des Zahnhalteapparats beim Patienten besteht. Durch die starke Sedimentation und Separation der Füllstoffe in den Einzelkomponenten A und B (Beispiel 16 und 17) während der Lagerzeit, sind auch die geforderten Eigenschaften der gemischten Komponenten nicht mehr sichergestellt. Mechanische und rheologische Eigenschaften sowie das Abbindeverhalten des Abformmaterials können nach Lagerung durch die Sedimentation der Einzelkomponeten stark beeinträchtigt sein.
Das rheologische Verhalten des Abformmaterials nach dem Lagertest von 1 Woche bei 60°C ist durch ein der Mischungsviskosität auf 10,2 Pas und der Thixotropie (Thixotropieindex 1,1) gekennzeichnet. Die Abbindecharakteristik des Abformmaterials verändert sich während der Lagerzeit deutlich.
Dieses Beispiel verdeutlicht, dass ein Zahnabformmaterial für den neuen Abformlöffel bei Verwendung von ausschließlich nicht verstärkenden Füllstoffen, nicht geeignet ist. Insbesondere das Verhalten im Lagertest hinsichtlich Sedimentation, sowie die hohe Shore A - Härte machen einen Einsatz für die neue Abformtechnik unmöglich.

### Beispiel 10 (Vergleichsbeispiel)

Komponente A eines Zahnabformmaterials, das als Füllstoffe eine Kombination aus einer hohen Konzentration an verstärkenden und einer geringen Konzentration an nicht verstärkenden Füllstoffen enthält.

In einem Vakuummischer werden 70 Teile eines vinylendgestoppten Polydimethylsiloxans mit einer Viskosität von 1000 Pas bei 20 °C mit 1 Teil eines Platinkatalysators vom Karstedt-Typ mit einem Gehalt an reinem Platin von 1% mit 24 Teilen einer hydrophobierten (mit Trimethylsilylgruppen oberflächenbehandelten) hochdispersen Kieselsäure mit einer BET-Oberfläche von 140m²/g und 5 Teilen eines mit Trimethylsilylgruppen oberflächenbehandelten Siliciumdioxid Füllstoffs (Quarz) mit einer BET - Oberfläche von 1 m²/g und einer mittleren Korngröße von 10 µm für 60 Minuten unter Vakuum homogen gemischt. Man erhält eine dünnfließende Paste mit einer Viskosität von 9,5 Pas, die eine geringe Thixotropie aufweist (Thixotropieindex 1,0). Die Paste zeigt während der Lagerzeit von 1 Woche 60°C eine geringe Sedimentation des Füllstoffs, jedoch keine Separation des Siliconpolymers. Weiterhin zeigt sich im Lagertest eine deutliche Erhöhung der Viskosität auf 13,4 Pas und eine gleichbleibende Thixotropie (Thixotropieindex 1,0).

### Beispiel 11 (Vergleichsbeispiel)

Komponente B eines Zahnabformmaterials, das als Füllstoffe eine Kombination aus einer hohen Konzentration an verstärkenden und einer geringen Konzentration an nicht verstärkenden Füllstoffen enthält. In einem Vakkuummischer werden 47 Teile eines vinylendgestoppten Polydimethylsiloxans mit einer Viskosität von 1000 mPas bei 20°C mit 25 Teilen eines Polymethylhydrogensiloxans mit einer Viskosität von 200 mPas bei 20°C und einem SiH-Gehalt von 1,8 mmol/g mit 23 Teilen einer hydrophobierten (mit Trimethylsilylgruppen oberflächenbehandelte) hochdispersen Kieselsäure mit einer BET - Oberfläche von 140 m²/g, 5 Teile eines mit Trimethylsilylgruppen oberflächenbehandelten Siliciumdioxid - Füllstoffs (Quarz) mit einer BET - Oberfläche von < 1 m²/g und einer mittleren Korngröße von 10 µm, 0,02 Teile eines Lebensmittelfarbstoffs und 0,013 Teile Divinyltetramethyldisiloxan für 60 Minuten unter Vakuum homogen gemischt.

Man erhält eine dünnfließende Paste mit einer Viskosität von 9,8 Pas, deren Thixotropieindex 1,0 ist. Die Paste zeigt während der Lagerzeit eine geringe Sedimentation der Füllstoffe, jedoch keine Separation des Siliconpolymere. Weiterhin zeigt sich im Lagertest eine deutliche Erhöhung der Viskosität auf 17,9 Pas und der Thixotropie.

### Beispiel 12 (Vergleichsbeispiel)

Mischung der Komponente A aus Beispiel 10 und der Komponente B aus Beispiel 11.

50 Teile der Komponente A aus Beispiel 10 und 50 Teile der Komponente B aus Beispiel 12 werden aus einer Doppelkammerkartusche über einen statischen Mischer ausgedrückt und homogen gemischt.

Man erhält eine dünnfließende Paste mit einer Mischungsviskosität 9,2 Pas, die eine geringe Thixotropie aufweist (Thixotropieindex 1,1).
Die Paste besitzt bei 35°C eine Verarbeitungszeit von 60 Sekunden und ist bei 35°C nach 240 Sekunden vollständig ausgehärtet. Man erhält nach vollständiger Aushärtung elastische Formkörper mit einer Shore A - Härte von 31.Die Rückstellung nach der Verformung nach ISO 4823 liegt bei 99,4 %. Die Verformung unter Druck nach ISO 4823 liegt bei 6,4 %. Die Reißfestigkeit und Weiterreißfestigkeit liegt bei 135 N/cm² bzw. 2,59 N/mm.

Die Reißfestigkeiten sind für ein Zahnabformmaterial für den neuen Abformlöffel nicht ausreichend. Bei Mundentnahme und Entnahme des Abdrucks aus dem Abformlöffel besteht die Gefahr, daß das Material einreißt oder bricht. Die Shore A - Härte ist ausreichend.
Das rheologische Verhalten des Abformmaterials nach dem Lagertest von 1 Woche bei 60 °C ist durch ein Ansteigen der Mischungsviskosität auf 10,7 Pas und eine gleichbleibende Thixotropie (Thixotropieindex 1,0) gekennzeichnet.
Die Abbindecharakteristik des Abformmaterials verändert sich während der Lagerzeit nicht.

Dieses Beispiel verdeutlicht, daß ein Zahnabformmaterial für den neuen Abformlöffel bei Verwendung einer Füllstoffkombination mit niedrigen Konzentrationen an nicht verstärkenden Füllstoffen und hohen Konzentrationen an verstärkenden Füllstoffen nicht geeignet ist. Die Reißfestigkeit der ausgehärteten Abformmasse ist zu niedrig und lässt deshalb einen Einsatz im neuen Abformlöffel nicht zu.

### Beispiel 13 (Vergleichsbeispiel)

Komponente A eines Zahnabformmaterials, das als Füllstoffe eine Kombination aus einer mittleren Konzentration an verstärkenden und einer mittleren Konzentration an nicht verstärkenden Füllstoffen enthält.

In einem Vakuummischer werden 51 Teile eines vinylendgestoppten Polydimethylsiloxans mit einer Viskosität von 1000 mPas bei 20°C mit 1 Teil eines Platinkatalysators vom Karstedt --Typ mit einem Gehalt an reinem Platin von 1% mit 11 Teilen einer hydrophobierten (mit Trimethylsilylgruppen oberflächenbehandelte) hochdispersen Kieselsäure mit einer BET-Oberfläche von 140 m²/g und 37 Teilen eines mit Trimethylsilylgruppen oberflächenbehandelten Siliciumdioxid Füllstoffs (Quarz) mit einer BET - Oberfläche von < 1m²/g und einer mittleren Korngröße von 10 µm für 60 Minuten unter Vakuum homogen gemischt.

Man erhält eine dünnfließende Paste mit einer Viskosität von 9,1 Pas, deren Thixotropieindex 1,1 beträgt. Die Paste zeigt während der Lagerzeit von 1 Woche 60°C eine mittlere Sedimentation des Füllstoffs und Separation des Siliconpolymers. Weiterhin zeigt sich im Lagertest eine deutliche Erhöhung der Viskosität auf 18,5 Pas und eine gleichbleibende Thixotropie.

### Beispiel 14 (Vergleichsbeispiel)

Komponente B eines Zahnabformmaterials, das als Füllstoff eine Kombination aus einer mittleren Konzentration an verstärkenden und einer mittleren Konzentration an nicht verstärkenden Füllstoffen enthält.

In einen Vakuummischer werden 41 Teile eines vinylendgestoppten Polydimethylsiloxans mit einer Viskosität von 200 mPas bei 20°C mit 13 Teilen eines Polymethylhydrogensiloxans, SiH-Gehalt von 4,3 mmol/g mit 10 Teilen einer hydrophobierten (mit Trimethylsilylgruppen oberflächenbehandelten) hochdispersen Kieselsäure mit einer BET-Oberfläche von 140 m²/g, 36 Teilen eines mit Trimethylsilylgruppen oberflächenbehandelten Siliciumdioxid Füllstoffs (Quarz) mit einer BET - Oberfläche von 1 m²/g und einer mittleren Korngröße von 10 µm und 0,01 Teile eines Lebensmittelfarbstoffs für 60 Minuten unter Vakuum homogen gemischt.

Man erhält eine dünnfließende Paste mit einer Viskosität von 7,6 Pas, deren Thixotropieindex 1,2 beträgt. Die Paste zeigt während der Lagerzeit von 1 Woche 60 °C eine geringe Sedimentation des Füllstoffs und Separation des Siliconpolymers. Weiterhin zeigt sich im Lagertest eine deutliche Erhöhung der Viskosität auf 12,0 Pas und eine gleichbleibende Thixotropie.

### Beispiel 15 (Vergleichsbeispiel)

Mischung der Komponente A aus Beispiel 13 und der Komponente B aus Beispiel 14.

50 Teile der Komponente A aus Beispiel 13 und 50 Teile der Komponente B aus Beispiel 14 werden aus einer Doppelkammerkartusche über einen statischen Mischer ausgedrückt und homogen gemischt.

Man erhält eine dünnfließende Paste mit einer Mischungsviskosität von 9,5 Pas, deren Thixotropieindex 1,0 beträgt. Die Paste besitzt bei 35 °C eine Verarbeitungszeit von 40 Sekunden und ist bei 35°C nach 240 Sekunden vollständig ausgehärtet. Man erhält nach vollständiger Aushärtung relativ harte, wenig elastische Formkörper. Die Rückstellung nach der Verformung liegt nach ISO 4823 bei 99,4 %.
Die Verformung unter Druck liegt nach ISO 4823 bei 3,3 %. Die Shore A-Härte beträgt 54. Die Reiß- und Weiterreißfestigkeit liegt bei 203 N/cm² bzw. 0,64 N/mm. Die Reißfestigkeiten sind für eine Zahnabformung mit dem neuen Abformlöffel gut geeignet, jedoch ist die Shore A-Härte etwas zu hoch.
Das rheologische Verhalten des Abformmaterials nach dem Lagertest von 1 Woche 60°C ist durch ein Ansteigen der Mischungsviskosität auf 13,9 Pas und der Thixotropie (Thixotropieindex 1,4) gekennzeichnet.
Die Abbindecharakteristik des Abformmaterials verändert sich während der Lagerzeit nicht. Durch die starke Sedimentation und Separation der Füllstoffe in den Einzelkomponenten A und B (Beispiel 13 und 14) während der Lagerzeit, sind auch die geforderten Eigenschaften der gemischten Komponenten nicht mehr sichergestellt.
Mechanische und rheologische Eigenschaften sowie das Abbindeverhalten des Abformmaterials können nach Lagerung durch die Sedimentation der Einzelkomponenten stark beeinträchtigt sein.

Dieses Beispiel verdeutlicht, dass ein Zahnabformmaterial für den neuen Abformlöffel bei Verwendung einer Füllstoffkombination mit mittleren Konzentrationen an verstärkenden und nicht verstärkenden Füllstoffen nicht geeignet ist da diese Formulierung eine deutliche Sedimentation der Füllstoffe aufweist und somit eine Lagerstabilität nicht gegeben ist. Das Fließverhalten ist aufgrund der deutlichen Thixotropie und dem Viskositäts- und Thixotropieanstieg nach Lagerung nicht für einen Einsatz im neuen Abformlöffel geeignet.

### Beispiel 16 (Vergleichsbeispiel)

Komponente A eines Zahnabformmaterials, das als Füllstoffe eine Kombination aus einer niedrigen Konzentration an verstärkenden Füllstoffen und eine hohe Konzentration an nicht verstärkenden Füllstoffen, enthält. In einem Vakuummischer werden 47 Teile eines vinylendgestoppten Polydimethylsiloxans mit einer Viskosität von 1000 mPas bei 20°C mit 1 Teil eines Platinkatalysators vom Karstedt - Typ mit einem Gehalt an reinem Platin von 1% mit 4 Teilen einer hydrophobierten, hochdispersen Kieselsäure mit einer BET- Oberfläche von 140 m²/g und 48 Teilen eines Trimethylsilylgruppen oberflächenbehandelten Siliciumdioxid - Füllstoffs (Quarz) mit einer BET - Oberfläche von < 1m²/g und einer mittleren Korngröße von 10 µm für 60 Minuten unter Vakuum homogen gemischt.

Man erhält eine dünnfließende Paste mit einer Viskosität von 9,0 Pas, deren Thixotropieindex 1,0 beträgt. Die Paste zeigt während der Lagerzeit von 1 Woche 60°C eine starke Sedimentation der Füllstoffe und Separation des Siliconpolymers. Weiterhin zeigt sich im Lagertest eine Erhöhung der Viskosität auf 11,0 Pas und der Thixotropie ( Thixotropieindex 1,1).

### Beispiel 17 (Vergleichsbeispiel)

Komponente B eines Zahnabformmaterials, das als Füllstoffe eine Kombination aus einer niedrigen Konzentration an verstärkenden Füllstoffen und eine hohe Konzentration an nicht verstärkenden Füllstoffen, enthält. In einem Vakuummischer werden 26 Teile eines vinylendgestoppten Polydimethylsiloxans mit einer Viskosität von 1000 mPas bei 20°C mit 16 Teilen eines Polymethylhydrogensiloxans mit einer Viskosität von 200 mPas bei 20°C und einem SiH-Gehalt von 1,8 mmol/g mit 4 Teilen einer hydrophobierten (mit Trimethylsilylgruppen oberflächenbehandelten) hochdispersen Kieselsäure mit einer BET-Oberfläche von 140 m²/g, 54 Teilen eines mit Trimethylsilylgruppen oberflächenbehandelten Siliciumdioxid - Füllstoffs (Quarz) mit einer BET - Oberfläche von 1m²/g und einer mittleren Korngröße von 10 µm, 0,02 Teile eines Lebensmittelfarbstoffs und 0,02 Teile Divinyltetramethyldisiloxan für 60 Minuten unter Vakuum homogen gemischt.

Man erhält eine dünnfließende Paste mit einer Viskosität von 9,9 Pas, deren Thixotropieindex 1,3 beträgt. Die Paste zeigt während der Lagerzeit eine starke Sedimentation der Füllstoffe und eine starke Separation der Siliconpolymere. Weiterhin zeigt sich im Lagertest eine deutliche Erhöhung der Viskosität auf 15 Pas und der Thixotropie ( Thixotropieindex 1,5).

### Beispiel 18 (Vergleichsbeispiel)

Mischung der Komponente A aus Beispiel 16 und der Komponente B aus Beispiel 17. 50 Teile der Komponente A aus Beispiel 16 und 50 Teile der Komponente B aus Beispiel 17 werden aus einer Doppelkammerkartusche über einen statischen Mischer ausgedruckt und homogen gemischt.

Man erhält eine dünnfließende Paste mit einer Viskosität von 8,5 Pas, deren Thixotropieindex 1,1 beträgt. Die Paste besitzt bei 35 °C eine Verarbeitungszeit von 60 s und ist bei 35°C nach 240 Sekunden vollständig ausgehärtet. Man erhält nach vollständiger Aushärtung elastische Formkörper mit einer Shore A-Härte von 57.

Die Rückstellung nach der Verformung nach ISO 4823 liegt bei 99,70 %. Die Verformung unter Druck liegt nach ISO 4823 bei 2,7 %. Die Reiß- und Weiterreißfestigkeit liegt bei 272 N/cm² bzw. 0,73 N/mm. Die Reißfestigkeiten und Shore A-Härte sind für eine Zahnabformung mit dem neuen Abformlöffel gut geeignet.
Das rheologische Verhalten der Abformmasse nach dem Lagertest von 1 Woche bei 60°C ist durch ein Ansteigen der Mischungsviskosität auf 12,4 Pas und eine gleichbleibende Thixotropie (Thixotropieindex 1,1) gekennzeichnet. Die Abbindezeit des Abformmaterials verkürzt sich während der Lagerzeit auf 185 s.

Durch die starke Sedimentaion und Separation der Füllstoffe in den Einzelkomponenten A und B (Beispiel 16 und 17) während der Lagerzeit, sind auch die geforderten Eigenschaften der gemischten Komponenten nicht mehr sichergestellt.
Mechanische und rheologische Eigenschaften sowie das Abbindeverhalten des Abformmaterials werden nach Lagerung durch die Sedimentation der Einzelkomponenten stark beeinträchtigt sein.

Dieses Beispiel verdeutlicht, dass ein Zahnabformmaterial für den neuen Abformlöffel bei Verwendung einer Füllstoffkombination mit niedrigen Konzentrationen an verstärkenden Füllstoffen und hohen Konzentrationen an nicht verstärkenden Füllstoffen nicht geeignet ist.

### Beispiel 19 (Vergleichsbeispiel)

Komponente A eines Zahnabformmaterials, das als Füllstoffe eine Kombination aus einer niedrigen Konzentration an verstärkenden Füllstoffen (BET-Oberfläche 200 m²/g) und eine hohe Konzentration an nicht verstärkenden Füllstoffen enthält.

In einem Vakuummischer werden 54 Teile eines vinylendgestoppten Polydimethylsiloxans mit einer Viskosität von 1000 mPas bei 20°C mit 1 Teil eines Platinkatalysators vom Karstedt - Typ mit einem Gehalt an reinem Platin von 1% mit 4 Teilen einer hydrophobierten (mit Trimethylsilylgruppen oberflächenbehandelten) hochdispersen Kieselsäure mit einer BET- Oberfläche von 200 m²/g und 41 Teilen eines Trimethylsilylgruppen oberflächenbehandelten Siliciumdioxid - Füllstoffs (Quarz) mit einer BET - Oberfläche von < 1m²/g und einer mittleren Korngröße von 10 µm für 60 Minuten unter Vakuum homogen gemischt.

Man erhält eine dünnfließende Paste mit einer Viskosität von 8,5 Pas, deren Thixotropieindex 1,1 beträgt. Die Paste zeigt während der Lagerzeit von 1 Woche 60°C eine starke Sedimentation der Füllstoffe und Separation des Siliconpolymers. Weiterhin zeigt sich im Lagertest eine deutliche Erhöhung der Viskosität auf 8,8 Pas und eine gleichbleibende Thixotropie.

### Beispiel 20 (Vergleichsbeispiel)

Komponente B eines Zahnabformmaterials, das als Füllstoffe eine Kombination aus einer niedrigen Konzentration an verstärkenden Füllstoffen (BET-Oberfläche 200 m²/g) und eine hohe Konzentration an nicht verstärkenden Füllstoffen, enthält.

In einem Vakuummischer werden 34 Teile eines vinylendgestoppten Polydimethylsiloxans mit einer Viskosität von 1000 mPas bei 20°C mit 20 Teilen eines Polymethylhydrogensiloxans mit einer Viskosität von 200 mPas bei 20°C und einem SiH-Gehalt von 1,8 mmol/g mit 4 Teilen einer hydrophobierten (mit Trimethylsilylgruppen oberflächenbehandelten) hochdispersen Kieselsäure mit einer BET-Oberfläche von 200 m²/g, 42 Teilen eines mit Trimethylsilylgruppen oberflächenbehandelten Siliciumdioxid - Füllstoffs (Quarz) mit einer BET - Oberfläche von <1m²/g und einer mittleren Korngröße von 10 µm, 0,02 Teile eines Lebensmittelfarbstoffs und 0,02 Teile Divinyltetramethyldisiloxan für 60 Minuten unter Vakuum homogen gemischt.

Man erhält eine dünnfließende Paste mit einer Viskosität von 9,4 Pas, deren Thixotropieindex 1,2 beträgt. Die Paste zeigt während der Lagerzeit eine starke Sedimentation der Füllstoffe und eine starke Separation der Siliconpolymere. Weiterhin zeigt sich im Lagertest eine deutliche Erhöhung der Viskosität auf 13,4 Pas und der Thixotropie ( Thixotropieindex 1,7).

### Beispiel 21 (Vergleichsbeispiel)

Mischung der Komponente A aus Beispiel 19 und der Komponente B aus Beispiel 20.

50 Teile der Komponente A aus Beispiel 19 und 50 Teile der Komponente B aus Beispiel 20 werden aus einer Doppelkammerkartusche über einen statischen Mischer ausgedrückt und homogen gemischt.

Man erhält eine dünnfließende Paste mit einer Viskosität von 6,4 Pas, deren Thixotropieindex 1,1 beträgt. Die Paste besitzt bei 35 °C eine Verarbeitungszeit von 60 s und ist bei 35°C nach 230 Sekunden vollständig ausgehärtet. Man erhält nach vollständiger Aushärtung elastische Formkörper mit einer Shore A-Härte von 51.

Die Rückstellung nach der Verformung nach ISO 4823 liegt bei 99,60 %. Die Verformung unter Druck liegt nach ISO 4823 bei 3,4 %. Die Reiß- und Weiterreißfestigkeit liegt bei 170 N/cm² bzw. 0,59 N/mm. Die Reißfestigkeiten und Shore A-Härte sind direkt nach Herstellung der Abformmasse für eine Zahnabformung mit dem neuen Abformlöffel gut geeignet. Das rheologische Verhalten der Abformmasse nach dem Lagertest von 1 Woche bei 60°C ist durch ein Ansteigen der Mischungsviskosität auf 7,6 Pas und der Thixotropie gekennzeichnet. Das Abbindeende des Abformmaterials beschleunigt sich während der Lagerzeit auf 175 Sekunden.
Durch die starke Sedimentation und Separation der Füllstoffe in den Einzelkomponenten A und B (Beispiel 19 und 20) in der Lagerzeit, sind auch die geforderten Eigenschaften der gemischten Komponenten nicht mehr sichergestellt.
Mechanische und rheologische Eigenschaften sowie das Abbindeverhalten des Abformmaterials werden nach Lagerung durch die Sedimentation der Einzelkomponenten stark beeinträchtigt sein.

Dieses Beispiel verdeutlicht, dass ein Zahnabformmaterial für den neuen Abformlöffel bei Verwendung einer Füllstoffkombination mit niedrigen Konzentrationen an verstärkenden Füllstoffen (BET - Oberfläche 200 m²/g) und hohen Konzentrationen an nicht verstärkenden Füllstoffen nicht geeignet ist.

### Beispiel 22 (Vergleichsbeispiel)

Komponente A eines Zahnabformmaterials, das als Füllstoff nur verstärkenden Füllstoff (BET-Oberfläche 200 m²/g) enthält.

In einem Vakuummischer werden 91 Teile eines vinylendgestoppten Polydimethylsiloxans mit einer Viskosität von 1,0 Pas bei 20°C mit 1 Teil eines Platinkatalysators vom Karstedt - Typ mit einem Gehalt an reinem Platin von 1% mit 8 Teilen einer hydrophobierten, (mit Trimethylsilylgruppen oberflächenbehandelten) hochdispersen Kieselsäure mit einer BET - Oberfläche von 200 m²/g für 60 Minuten unter Vakuum homogen gemischt.

Man erhält eine dünnfließende Paste mit einer Viskosität von 9,2 Pas, deren Thixotropieindex 1,0 beträgt. Die Paste zeigt während der Lagerzeit von einer Woche 60 °C nahezu keine Veränderung von Viskosität und Thixotropie auf.
Die Paste weist während der Lagerzeit naturgemäß keine Sedimentation oder Separation auf.

### Beispiel 23 (Vergleichsbeispiel)

Komponente B eines Zahnabformmaterials, das als Füllstoff nur verstärkenden Füllstoff (BET-Oberfläche 200 m²/g) enthält.

In einem Vakuummischer werden 58 Teile eines vinylendgestoppten Polydimethylsiloxans mit einer Viskosität von 1,0 Pas bei 20 °C mit 32 Teilen eines Polymethylhydrogensiloxans mit einer Viskosität von 0,2 Pas und einem SiH-Gehalt von 1,8 mmol/g, mit 10 Teilen einer hydrophobierten, (mit Trimethylsilylgruppen oberflächenbehandelten) hochdispersen Kieselsäure mit einer BET - Oberfläche von 200 m²/g und 0,01 Teilen eines Lebensmittelfarbstoffs für 60 Minuten unter Vakuum homogen gemischt. Man erhält eine dünnfließende Paste mit einer Viskosität von 9,1 Pas, deren Thixotropieindex 1,0 beträgt. Die Paste zeigt nach der Lagerzeit von einer Woche 60°C nahezu keine Veränderung von Viskosität und Thixotropie auf.
Die Paste weist während der Lagerzeit keine Sedimentation oder Separation auf.

### Beispiel 24 (Vergleichsbeispiel)

Mischung der Komponente A aus Beispiel 22 und der Komponente B aus Beispiel 23.

50 Teile der Komponente A aus Beispiel 22 und 50 Teile der Komponente B aus Beispiel 23 werden aus einer Doppelkammerkartusche über einen statischen Mischer ausgedrückt und homogen gemischt. Man erhält eine dünnfließende Paste mit einer Mischungsviskosität von 7,1 Pas, deren Thixotropieindex 1,1 beträgt. Die Paste besitzt bei 35 °C eine Verarbeitungszeit von 60 s und ist bei 35 °C nach 240 s vollständig ausgehärtet.

Man erhält nach vollständiger Aushärtung transparente, weiche, elastische Formkörper. Die Rückstellung nach der Verformung liegt bei 99,70 % (ISO 4823). Die Verformung unter Druck nach ISO 4823 liegt bei 6,4 %. Die Shore A-Härte liegt bei 31. Die Reiß- und Weiterreißfestigkeit liegt bei 40 N/cm² bzw. 0,32 N/mm.

Diese mechanischen Eigenschaften sind für ein Zahnabformmaterial für den neuen Abformlöffel nicht ausreichend. Bei Mundentnahme und Entnahme des Abdrucks aus dem Abformlöffel besteht die Gefahr, dass das Material bricht und einreißt.
Das rheologische Verhalten des Abformmaterials nach dem Lagertest zeigt eine Mischungsviskosität von 7,4 Pas und einen Thixotropieindex 1,0 nach der Lagerzeit (1 Woche 60°C). Die Abbindecharakteristik des Abformmaterials verändert sich während der Lagerzeit auf eine Abbindezeit von 280 s.

### Beispiel 25 (erfindungsgemäß)

Komponente A eines erfindungsgemäßen Zahnabformmaterials für den neuen Abformlöffel mit einer Mischungsviskosität von ca. 7,5 Pas.

In einem Vakuummischer werden 72 Teile eines vinylendgestoppten Polydimethylsiloxans mit einer Viskosität von 1000 mPas bei 20°C mit 1 Teil eines Platinkatalysators vom Karstedt - Typ mit einem Gehalt an reinem Platin von 1% mit 25 Teilen einer hydrophilen, naßgefällten Kieselsäure, die einen Anteil an Calciumoxid von 6% aufweist und eine BET-Oberfläche von 35m²/g besitzt und durch Trocknung über 48 Stunden bei 130°C auf einen Restwassergehalt von 0,3 % gebracht wurde und 2 Teilen einer hydrophobierten, (mit Trimethylsilylgruppen oberflächenbehandelten) hochdispersen Kieselsäure mit einer BET - Oberfläche von 140 m²/g für 60 Minuten unter Vakuum homogen gemischt.

Man erhält eine dünnfließende Paste mit einer Viskosität von 6,7 Pas, dern Thixotropieindex 1,1 beträgt. Die Paste zeigt während der Lagerzeit von 3 Wochen bei 60°C keine Sedimentation der Füllstoffe und keine Separation der Siliconpolymere.
Im Lagertest steigt die Viskosität auf 8,1 Pas und die Thixotropie an (Thixotropieindex 1,4).

### Beispiel 26 (erfindungsgemäß)

Komponente B eines erfindungsgemäßen Zahnabformmaterials für den neuen Abformlöffel mit einer Mischungsviskosität von ca. 7,5 Pas.

In einem Vakuummischer werden 47 Teile eines vinylendgestoppten Polydimethylsiloxans mit einer Viskosität von 1000 mPas bei 20°C mit 26 Teilen eines Polymethyl-hydrogensiloxans mit einer Viskosität von 200 mPas bei 20°C und einem SiH-Gehalt von 1,80 mmol/g, 25 Teilen einer hydrophilen, naßgefällten Kieselsäure, die einen Anteil an Calciumoxid von 6% aufweist und eine BET-Oberfläche von 35 m²/g besitzt und durch Trocknung über 48 Stunden bei 130°C auf einen Restwassergehalt von 0,3 % gebracht wurde, 2 Teilen einer hydrophobierten, (mit Trimethylsilylgruppen oberflächenbehandelten) hochdispersen Kieselsäure mit einer BET - Oberfläche von 140m²/g für 60 Minuten unter Vakuum homogen gemischt.

Man erhält eine dünnfließende Paste mit einer Viskosität von 7,3 Pas, deren Thixotropieindex 1,0 beträgt. Die Paste zeigt während einer Lagerzeit von 3 Wochen bei 60°C keine Sedimentation der Füllstoffe und Separation der Siliconpolymere. Im Lagertest steigen die Viskosität auf 7,9 Pas und die Thixotropie leicht an (Thixotropieindex 1,1).

### Beispiel 27 (erfindungsgemäß)

Mischung aus Komponente A aus Beispiel 25 und Komponente B aus Beispiel 26.

50 Teile der Komponente A aus Beispiel 25 und 50 Teile der Komponente B aus Beispiel 26 werden aus einer Doppelkammerkartusche über einen statischen Mischer ausgedrückt und homogen gemischt.

Man erhält eine dünnfließende Paste mit einer Mischungsviskosität von 7,3 Pas, deren Thixotropieindex 1,1 beträgt. Die Paste besitzt bei 35°C eine Verarbeitungszeit von 60 s und ist bei 35°C nach 215 Sekunden vollständig ausgehärtet.

Man erhält nach vollständiger Aushärtung elastische Formkörper mit einer Shore A Härte von 41. Die Rückstellung nach der Verformung liegt bei 99,90% (ISO 4823). Die Verformung unter Druck nach ISO 4823 liegt bei 6,40 %. Die Reiß- und Weiterreißfestigkeit liegt bei 225 N/cm² bzw. 0,59 N/mm.

Die mechanischen Eigenschaften sind für eine Abformung mit dem neuen Abformlöffel sehr gut geeignet. Die Mundentnahme ist ohne Gefahr für den Zahnhalteapparat des Patienten schonend möglich. Das ausgehärtete Material zeigt auf Grund seiner hohen Reißfestigkeit kein unerwünschtes Einreißen bei Mundentnahme oder Entnahme des Abdrucks aus dem Abformlöffel. Das rheologische Verhalten der erfindungsgemäßen Abformmasse nach dem Lagertest zeigt eine unveränderte Thixotropie (Thixotropieindex 1,1). Auch das Abbindeverhalten der Abformmasse verändert sich während der Lagerzeit nicht.

Auf diese Weise ist sichergestellt, dass das sehr gute Fließverhalten und damit die Präzision der Abformergebnisse auch während der Lagerzeit des erfindungsgemäßen Abformmaterials erhalten bleibt.

Bei Anwendung des erfindungsgemäßen Abformmaterials im neuen Abformlöffel am Patienten erhält man hervorragende, hochpräzise Abformergebnisse.

Dieses Beispiel verdeutlicht, dass ein Zahnabformmaterial für den neuen Abformlöffel bei Verwendung des erfindungsgemäß einzusetzenden Füllstoffs mit einer BET-Oberfläche von 20 bis 50 m²/g hinsichtlich der rheologischen Eigenschaften, der mechanischen Eigenschaften, des kinetischen Verhaltens und des Verhaltens im Lagertest sehr gut geeignet ist.

### Beispiel 28 (erfindungsgemäß)

Komponente A eines Zahnabformmaterials für den neuen Abformlöffel, wobei der erfindungsgemäße Füllstoff ungetrocknet eingesetzt wurde.

In einem Vakuummischer werden 72 Teile eines vinylendgestoppten Polydimethylsiloxans mit einer Viskosität von 1000 mPas bei 20°C mit 1 Teil eines Platinkatalysators vom Karstedt - Typ mit einem Gehalt an reinem Platin von 1% mit 25 Teilen einer hydrophophilen, naßgefüllten Kieselsäure , die einen Anteil an Calciumoxid von 6 % aufweist, eine BET-Oberfläche von 35m²/g besitzt, die ungetrocknet eingesetzt wird und einen Wassergehalt von 6,0 % hat, 2 Teile einer hydrophobierten (mit Trimethylsilylgruppen oberflächenbehandelten) hochdispersen Kieselsäure mit einer BET - Oberfläche von 140 m²/g und für 60 Minuten unter Vakuum homogen gemischt. Man erhält eine dünnfließende Paste mit einer Viskosität von 6,7 Pas, deren Thixotropieindex 1,1 beträgt. Die Paste zeigt während der Lagerzeit von 1 Woche 60°C keine Sedimentation der Füllstoffe und keine Separation der Siliconpolymere.

Im Lagertest steigt die Viskosität auf 8,1 Pas und die Thixotropie an (Thixotropieindex 1,4).

Die Paste ist bereits nach Einlagerung für eine Woche bei 60°C durch Zersetzung des Platinkatalysators braun verfärbt. Dies ist auf die Anwesenheit des Wassers im ungetrockneten Füllstoff zurückzuführen.

### Beispiel 29 (erfindungsgemäß)

Komponente B eines erfindungsgemäßen Zahnabformmaterials für den neuen Abformlöffel.

In einem Vakuummischer werden 47 Teile eines vinylendgestoppten Polydimethylsiloxans mit einer Viskosität von 1000 mPas bei 20°C mit 26 Teilen eines Polymethylhydrogensiloxans mit einer Viskosität von 200 mPas bei 20°C und einem SiH - Gehalt von 1,80 mmol/g, 25 Teile einer hydrophilen, naßgefällten Kieselsäure, die einen Anteil an Calciumoxid von 6% aufweist und eine BET-Oberfläche von 35 m²/g und einen Wassergehalt von 6% besitzt und durch Trocknung über 48 Stunden bei 130°C auf einen Restwassergehalt von 0,3% gebracht wurde, 2 Teile einer hydrophobierten (mit Trimethylsilylgruppen oberflächenbehandelten) hochdispersen Kieselsäure mit einer BET - Oberfläche von 140 m²/g für 60 Minuten unter Vakuum homogen gemischt.

Man erhält eine dünnfließende Paste mit einer Viskosität von 7,3 Pas, deren Thixotropieindex 1,0 beträgt. Die Paste zeigt während einer Lagerzeit von 3 Wochen bei 60°C keine Sedimentation der Füllstoffe und Separation der Siliconpolymere. Im Lagertest steigen die Viskosität auf 7,9 Pas und die Thixotropie leicht an (Thixotropieindex 1,1).
Bereits nach einer Woche Einlagerung bei 60°C sind die als Lagergebinde verwendeten Aluminiumtuben deutlich aufgebläht. Dies ist auf Wasserstoffentwicklung durch Reaktion des SiH-Vernetzers mit dem im Füllstoff enthaltenen Wasser zurückzuführen.

### Beispiel 30 (erfindungsgemäß)

Mischung aus Komponente A aus Beispiel 28 und Komponente B aus Beispiel 29.

50 Teile der Komponente A aus Beispiel 28 und 50 Teile der Komponente B aus Beispiel 29 werden aus einer Doppelkammerkartusche über einen statischen Mischer ausgedrückt und homogen gemischt.

Man erhält eine dünnfließende Paste mit einer Mischungsviskosität von 7,3 Pas, deren Thixotropieindex 1,1 beträgt.

Die Abformmasse weist unmittelbar nach Herstellung dieselben positiven Eigenschaften auf wie das Abformmaterial nach Beispiel 27.

Jedoch weist das Abformmaterial auf Grund der in den Beispielen 28 und 29 beschriebenen Instabilitäten der Einzelkomponenten durch den hohen Wassergehalt des Füllstoffs eine starke Abbindeverzögerung gegenüber dem Ausgangswert von 215 s nach Einlagerung für eine Woche bei 60°C auf (Abbindeende 600 s).

Aus diesem Grund ist ein Einsatz des erfindungsgemäß einzusetzenden Füllstoffs in ungetrockneter Form für ein Abformmaterial für den neuen Abformlöffel nicht optimal.

### Beispiel 31 (erfindungsgemäß)

Komponente A eines erfindungsgemäßen Zahnabformmaterials für den neuen Abformlöffel mit einer Mischungsviskosität von ca. 40 Pas.

In einem Vakuumischer werden 72 Teile eines vinylendgestoppten Polydimethylsiloxans mit einer Viskosität von 7,0 Pas bei 20°C mit 1 Teil eines Platinkatalysators vom Karstedt - Typ mit einem Gehalt an reinem Platin von 1% mit 25 Teilen einer hydrophilen, naßgefällten Kieselsäure, die einen Anteil an Calciumoxid von 6% aufweist, eine BET-Oberfläche von 35m²/g besitzt und durch Trocknung bei 130°C über 48 Stunden auf einen Restwassergehalt von 0,3 % gebracht wurde und 2 Teilen einer hydrophobierten, (mit Trimethylsilylgruppen oberflächenbehandelten) hochdispersen Kieselsäure mit einer BET - Oberfläche von 140 m²/g für 60 Minuten unter Vakuum homogen gemischt.

Man erhält eine dünnfließende Paste mit einer Viskosität von 36,8 Pas, deren Thixotropieindex 1,2 beträgt. Die Paste zeigt während der Lagerzeit von 3 Wochen bei 60°C keine Sedimentation der Füllstoffe und keine Separation der Siliconpolymere.
Im Lagertest steigt die Viskosität auf 38,2 Pas und die Thixotropie bleibt nahezu unverändert (Thixotropieindex 1,2).

### Beispiel 32 (erfindungsgemäß)

Komponente B eines erfindungsgemäßen Zahnabformmaterials für den neuen Abformlöffel mit einer Mischungsviskosität von ca. 40 Pas.

In einem Vakuumischer werden 60 Teile eines vinylendgestoppten Polydimethylsiloxans mit einer Viskosität von 7,0 Pas bei 20°C mit 13 Teilen eines Polymethylhydrogensiloxans mit einem SiH-Gehalt von 1,8 mmol/g, 25 Teilen einer hydrophilen, naßgefällten Kieselsäure, die einen Anteil an Calciumoxid von 6 % aufweist, eine BET-Oberfläche von 35 m²/g besitzt und durch Trocknung über 48 Stunden bei 130°C auf einen Restwassergehalt von 0,3 % gebracht wird, 2 Teilen einer hydrophobierten, (mit Trimethylsilylgruppen oberflächenbehandelten) hochdispersen Kieselsäure mit einer BET - Oberfläche von 140m²/g für 60 Minuten unter Vakuum homogen gemischt.

Man erhält eine dünnfließende Paste mit einer Viskosität von 25,5 Pas, deren Thixotropieindex 1,1 beträgt. Die Paste zeigt während der Lagerzeit von 3 Wochen bei 60°C keine Sedimentation der Füllstoffe und keine Separation der Siliconpolymere.
Im Lagertest steigt die Viskosität auf 28,4 Pas und die Thixotropie leicht an (Thixotropieindex 1,2).

### Beispiel 33 (erfindungsgemäß)

Mischung aus Komponente A aus Beispiel 31 und Komponente B aus Beispiel 32 mit einer Mischungsviskosität von ca. 40 Pas. 50 Teile der Komponente A aus Beispiel 31 und 50 Teile der Komponente B aus Beispiel 32 werden aus einer Doppelkammerkartusche über einen statischen Mischer ausgedrückt und homogen gemischt.

Man erhält eine dünnfließende Paste mit einer Mischungsviskosität von 39,8 Pas, deren Thixotropieindex 1,1 beträgt.

Die Paste besitzt bei 35°C eine Verarbeitungszeit von 60 s und ist bei 35°C nach 180 Sekunden vollständig ausgehärtet.
Man erhält nach vollständiger Aushärtung elastische Formkörper mit einer Shore A Härte von 35. Die Rückstellung nach der Verformung unter Druck nach ISO 4823 liegt bei 4,60 %. Die Reiß- und Weiterreißfestigkeit liegt bei 174 N/cm² bzw. 2,09 N/mm.

Die mechanischen Eigenschaften sind für eine Abformung mit dem neuen Abformlöffel sehr gut geeignet. Die Mundentnahme ist ohne Gefahr für den Zahnhalteapparat des Patienten schonend möglich. Das ausgehärtete Material zeigt auf Grund seiner hohen Reißfestigkeit kein unerwünschtes Einreißen bei Mundentnahme oder Entnahme des Abdrucks aus dem Abformlöffel. Das rheologische Verhalten der erfindungsgemäßen Abformmasse nach dem Lagertest zeigt eine nahezu unveränderte Mischungsviskosität von 39,9 Pas und unveränderte Thixotropie (Thixotropieindex 1,1). Auch das Abbindeverhalten der Abformmasse verändert sich während der Lagerzeit nicht.

Auf diese Weise ist sichergestellt, dass das sehr gute Fließverhalten und damit der Präzision des Zahnabdrucks auch während der Lagerzeit des erfindungsgemäßen Abformmaterials erhalten bleibt.

Bei Anwendung des erfindungsgemäßen Abformmaterials im neuen Abformlöffel am Patienten erhält man gute, hochpräzise Abformergebnisse.

Dieses Beispiel verdeutlicht, dass ein Zahnabformmaterial mit einer Mischungsviskosität von 40 Pas für den neuen Abformlöffel bei Verwendung des erfindungsgemäß einzusetzenden Füllstoffs mit einer BET-Oberfläche von 20 bis 50 m²/g hinsichtlich der rheologischen Eigenschaften, des kinetischen Verhaltens und des Verhaltens im Lagertest gut geeignet ist. Im Vergleich zu Beispiel 27 erhält man aufgrund der höheren Mischungsviskosität von 40 Pas keine sehr guten, sondern nur gute Abformergebnisse. Diese Qualitätsabstufung zeigt sich in schwierigen Präparationssituationen, die sich in der zur Fließrichtung abgewandten Seite hinter voluminösen Zähnen befinden. Hier kann es zu einem unvollständigen Umfließen dieser schwer zugänglichen Bereiche kommen. Dies äußert sich durch Fehlstellen in der fertigen Abformung. Dieser Effekt tritt bei dem erfindungsgemäßen Beispiel 27 mit dem besonders bevorzugten Viskositätsbereich von 1 bis 10 Pas nicht auf.

### Beispiel 34 (Vergleichsbeispiel)

Komponente A eines handelsüblichen dünnfließenden Zahnabformmaterials, das für konventionelle Abformtechniken (Korrekturabformaterial) eingesetzt wird.

Die Komponente A liegt als dünnfließende Paste mit einer Viskosität von 10,9 Pas vor, die eine starke Thixotropie aufweist (Thixotropieindex 9,0). Die Paste zeigt während der Lagerzeit von einer Woche 60°C ein deutliches Ansteigen der Viskosität auf 23,0 Pas und noch eine starke Thixotropie (Thixotropieindex 5,7).
Die Paste zeigt während der Lagerzeit keine Sedimentation der Füllstoffe und keine Separation der Siliconpolymere.

### Beispiel 35 (Vergleichsbeispiel)

Komponente B eines handelsüblichen dünnfließenden Zahnabformmaterials (Korrekturabformmaterial), das für konventionelle Abformtechniken eingesetzt wird.

Die Komponente B liegt als dünnfließende Paste mit einer Viskosität von 16 Pas vor, die eine starke Thixotropie aufweist (Thixotropieindex 49,0). Die Paste zeigt während der Lagerzeit von einer Woche 60°C ein Ansteigen der Viskosität auf 20,0 Pas und der Thixotropie (Thixotropieindex 63,8).
Die Paste zeigt während der Lagerzeit keine Sedimentation der Füllstoffe und keine Separation der Siliconpolymere.

### Beispiel 36 (Vergleichsbeispiel)

Mischung aus Komponente A aus Beispiel 34 und Komponente B aus Beispiel 35 (handelsübliches Zahnabformmaterial für konventionelle Abformtechniken).
50 Teile der Komponente A aus Beispiel 34 und 50 Teile der Komponente B
aus Beispiel 35 werden aus einer Doppelkammerkartusche über einen statischen Mischer ausgedrückt und homogen gemischt.

Man erhält eine dünnfließende Paste mit einer Mischungsviskosität von 38,9 Pas, deren Thixotropieindex 1,7 beträgt.
Die Paste besitzt bei 35°C eine Verarbeitungszeit von 60 Sekunden und ist bei 35°C nach 150 s vollständig ausgehärtet. Man erhält nach Aushärtung elastische Formkörper mit einer Shore A-Härte von 46. Die Rückstellung nach der Verformung unter Druck liegt nach ISO 4823 bei 99,80 %. Die Verformung unter Druck liegt nach ISO 4823 bei 4,60%. Die Reiß- und Weiterreißfestigkeit liegt bei 200 N/cm² bzw. 2,09 N/mm. Die mechanischen Eigenschaften wären für eine Zahnabformung mit dem neuen Abformlöffel gut geeignet, jedoch verursachen die stark thixotropen Fließeigenschaften des handelsüblichen Zahnabformmaterials bei Anwendung im neuen Abformlöffel am Patienten beim Umfließen der Zähne in zur Fließrichtung abgewandten Bereichen Fließfahnen, die das Abformergebnis stark verfälschen. Ein Einsatz im neuen Abformlöffel ist nicht sinnvoll.

### Beispiel 37 (erfindungsgemäß)

Komponente B eines erfindungsgemäßen Zahnabformmaterials für den neuen Abformlöffel mit einer Mischungsviskosität von ca. 7,5 Pas.

In einem Vakuummischer werden 46 Teile eines vinylendgestoppten Polydimethylsiloxans mit einer Viskosität von 7000 mPas bei 20°C mit 20 Teilen eines Polymethyl-hydrogensiloxans mit einer Viskosität von 200 mPas bei 20°C und einem SiH-Gehalt von 1,80 mmol/g und 7 Teilen eines Polysiloxan-Polyether-Copolymers mit einer Viskosität von 200 mPas und einem Vinylgehalt von 0,15 mmol/g., 25 Teilen einer hydrophilen, naßgefällten Kieselsäure, die einen Anteil an Calciumoxid von 6% aufweist und eine BET-Oberfläche von 35 m²/g besitzt und durch Trocknung über 48 Stunden bei 130°C auf einen Restwassergehalt von 0,3 % gebracht wurde, 2 Teilen einer hydrophobierten, (mit Trimethylsilylgruppen oberflächenbehandelten) hochdispersen Kieselsäure mit einer BET - Oberfläche von 140m²/g für 60 Minuten unter Vakuum homogen gemischt.

Man erhält eine dünnfließende Paste mit einer Viskosität von 7,3 Pas, deren Thixotropieindex 1,0 beträgt. Die Paste zeigt während einer Lagerzeit von 3 Wochen bei 60°C keine Sedimentation der Füllstoffe und Separation der Siliconpolymere. Im Lagertest steigen die Viskosität auf 7,9 Pas und die Thixotropie leicht an (Thixotropieindex 1,1).

### Beispiel 38 (erfindungsgemäß)

Mischung aus Komponente A aus Beispiel 25 und Komponente B aus Beispiel 37.

50 Teile der Komponente A aus Beispiel 25 und 50 Teile der Komponente B aus Beispiel 37 werden aus einer Doppelkammerkartusche über einen statischen Mischer ausgedrückt und homogen gemischt.

Man erhält eine dünnfließende Paste mit einer Mischungsviskosität von 7,3 Pas, deren Thixotropieindex 1,1 beträgt. Die Paste besitzt bei 35°C eine Verarbeitungszeit von 60 s und ist bei 35°C nach 215 Sekunden vollständig ausgehärtet.

Man erhält nach vollständiger Aushärtung elastische Formkörper mit einer Shore A Härte von 41. Die Rückstellung nach der Verformung liegt bei 99,90% (ISO 4823). Die Verformung unter Druck nach ISO 4823 liegt bei 6,40 %. Die Reiß- und Weiterreißfestigkeit liegt bei 225 N/cm² bzw. 0,59 N/mm.

Die mechanischen Eigenschaften sind für eine Abformung mit dem neuen Abformlöffel sehr gut geeignet. Die Mundentnahme ist ohne Gefahr für den Zahnhalteapparat des Patienten schonend möglich. Das ausgehärtete Material zeigt auf Grund seiner hohen Reißfestigkeit kein unerwünschtes Einreißen bei Mundentnahme oder Entnahme des Abdrucks aus dem Abformlöffel. Das rheologische Verhalten der erfindungsgemäßen Abformmasse nach dem Lagertest zeigt eine unveränderte Thixotropie (Thixotropieindex 1,1). Auch das Abbindeverhalten der Abformmasse verändert sich während der Lagerzeit nicht.

Auf diese Weise ist sichergestellt, dass das sehr gute Fließverhalten und damit die Präzision der Abformergebnisse auch während der Lagerzeit des erfindungsgemäßen Abformmaterials erhalten bleibt.

Bei Anwendung des erfindungsgemäßen Abformmaterials im neuen Abformlöffel am Patienten erhält man hervorragende, hochpräzise Abformergebnisse.

Dieses Beispiel verdeutlicht, dass ein Zahnabformmaterial für den neuen Abformlöffel bei Verwendung des erfindungsgemäß einzusetzenden Füllstoffs mit einer BET-Oberfläche von 20 bis 50 m²/g hinsichtlich der rheologischen Eigenschaften, der mechanischen Eigenschaften, des kinetischen Verhaltens und des Verhaltens im Lagertest sehr gut geeignet ist.

Darüber hinaus zeigt dieses Beispiel, dass der erfindungsgemäße Füllstoff bei Einsatz eines Silicopolyethers seine herausragenden Eigenschaften hinsichtlich Sedimentations- und Separationsverhalten und Thixotropie der Mischung zur Geltung bringt. Weiterhin wird die an sich naturbedingte Entmischungsneigung eines Silicopolyethers in einer Silicopolymermatrix durch den erfindungsgemäßen Füllstoff verhindert. Der Einsatz des Silicopolyethers wiederum führt aufgrund seiner hydrophilen Eigenschaften zur ausgezeichneten Abformergebnissen während des Aushärtens im Patientenmund. Der Silicopolyether wird beim Aushärten des erfindungsgemäßen 2-Komponentenmaterial in das Netzwerk eingebaut und wird beim Desinfizieren des fertigen Abdrucks daher nicht ausgewaschen. Dies wiederum führt beim anschließenden Ausgießen mit wässrigem Gipsbrei zu einem guten Anfließen des Gips an den Abdruck und somit zu ausgezeichneten Modellqualitäten.

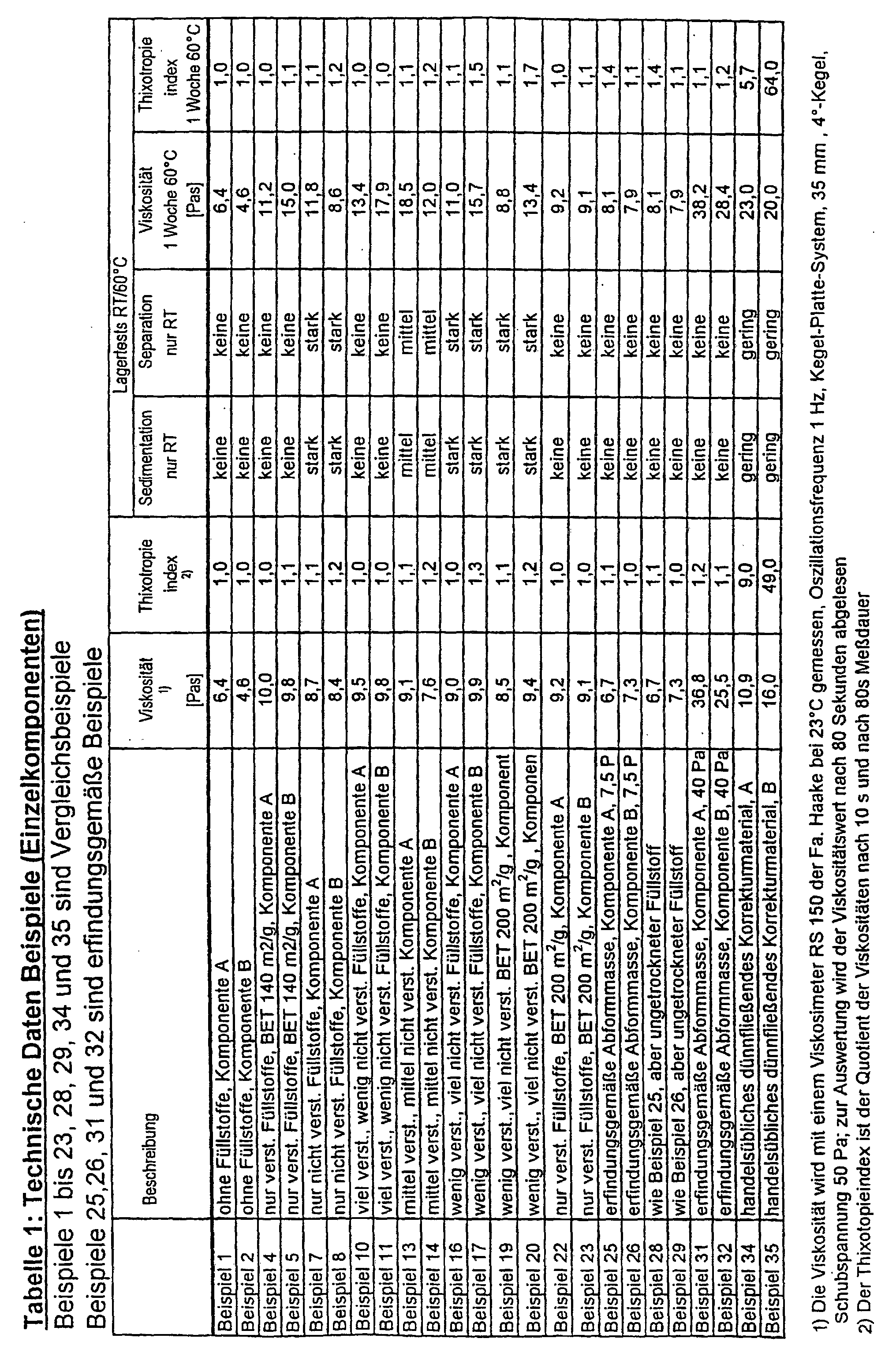

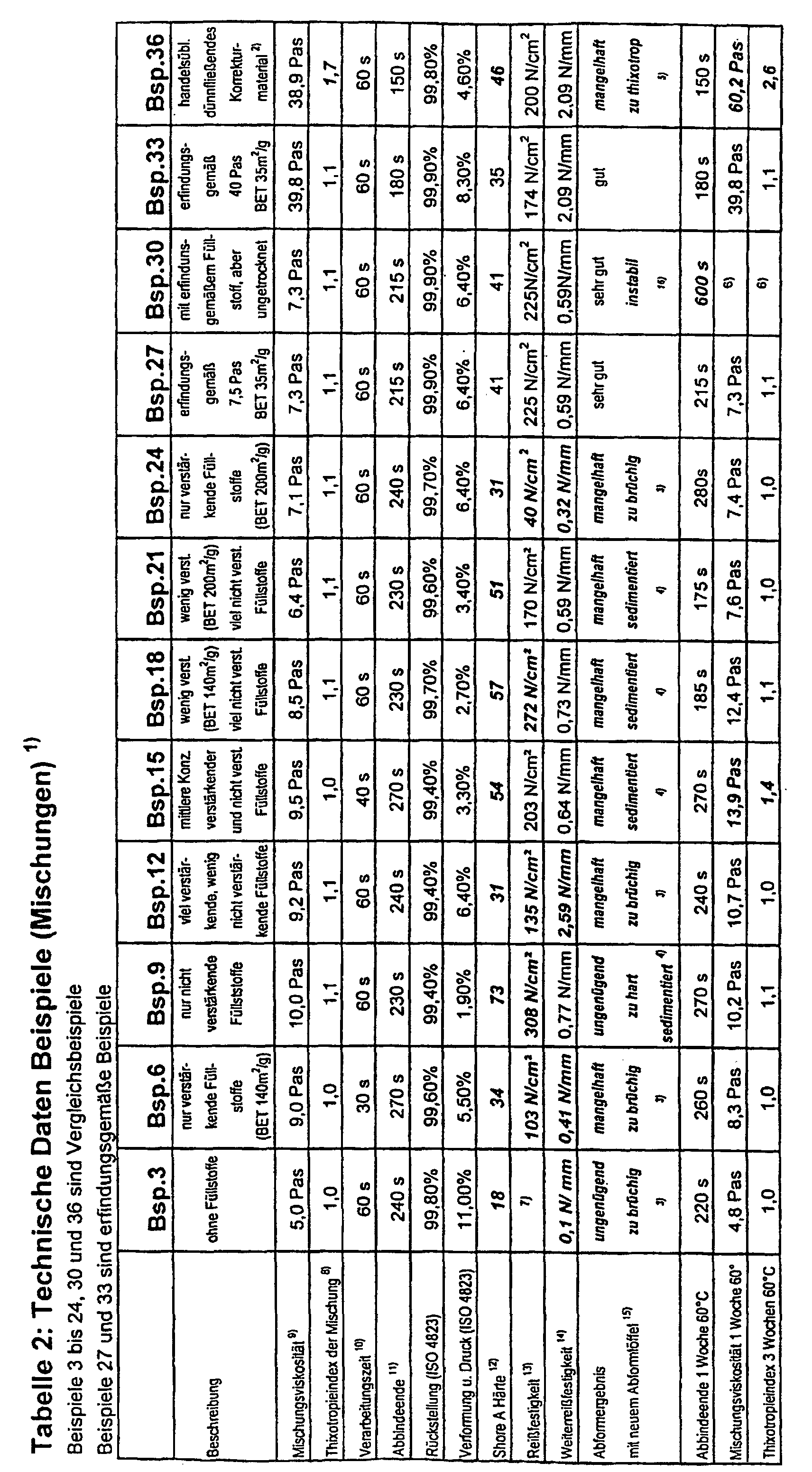

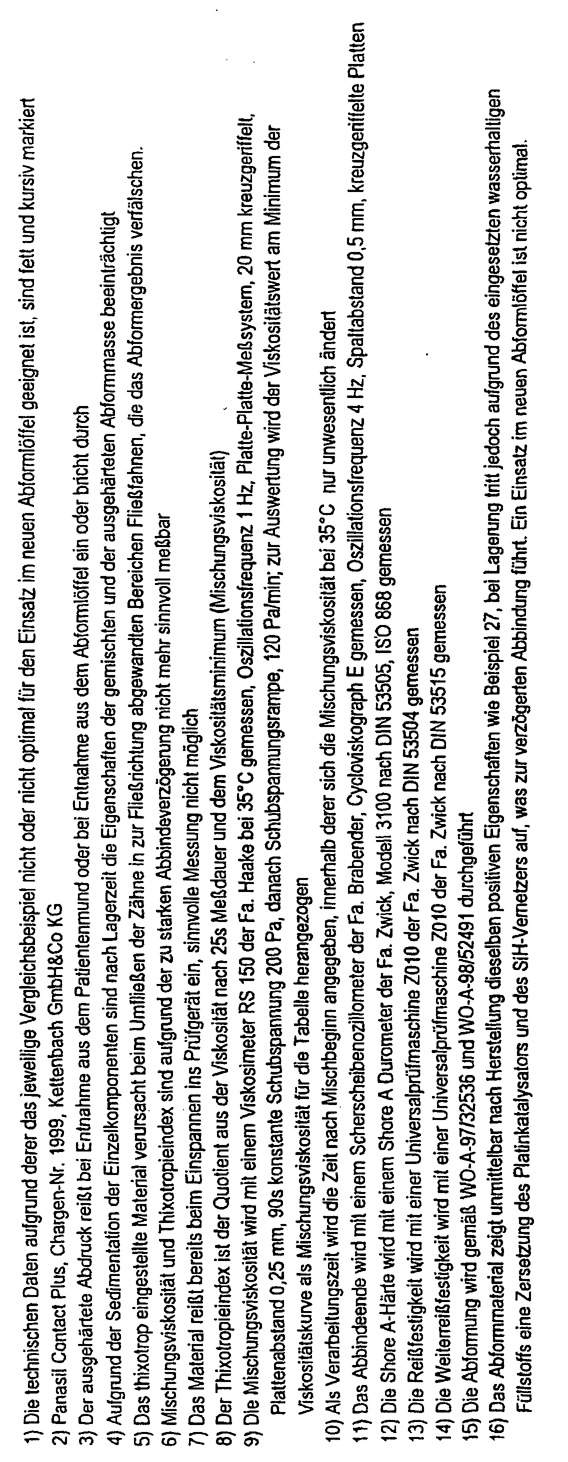

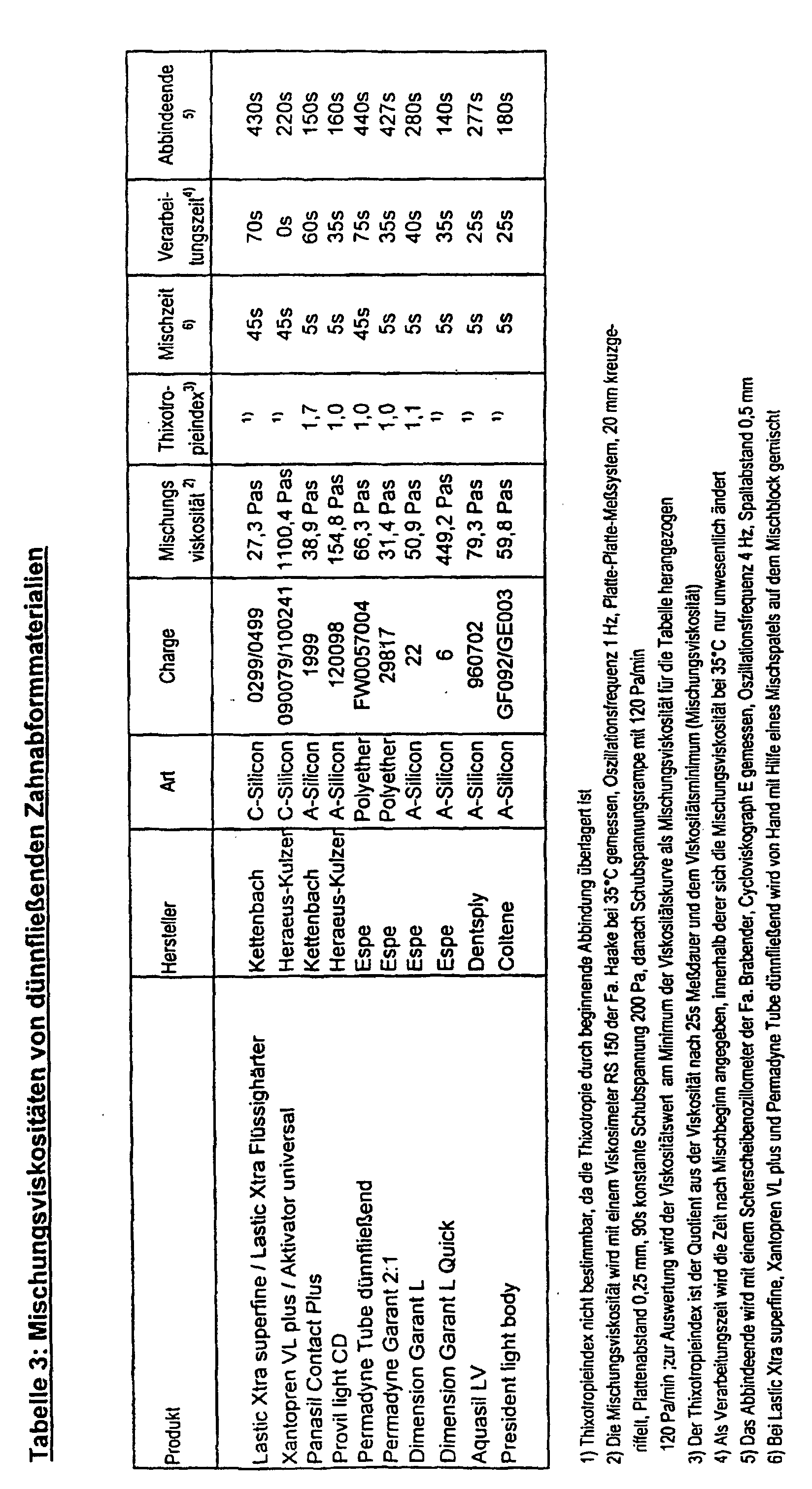

## Patentansprüche

1. Abformmaterial zur Verwendung mit einem Abformlöffel nach WO-A-97/32536 oder WO-A-98/52491 an Patienten, wobei das Abformmaterial härtbare Komponenten und mindestens einen ersten Füllstoff aufweist, **dadurch gekennzeichnet, dass** der mindestens erste Füllstoff eine BET Oberfläche von 20 bis 50 m²/g, vorzugsweise 30 bis 40 m²/g aufweist und in einer Menge von 20 bis 30 Gew.% vorliegt, wodurch das Abformmaterial einen Thixotropieindex von ≤ 1,1 aufweist und eine Viskosität von 1 bis 40 Pas, bevorzugt 1-10 Pas erreicht.

2. Abformmaterial nach Anspruch 1, **dadurch gekennzeichnet, dass** durch den Einsatz des ersten Füllstoffs während der Haltbarkeitsdauer von mindestens 18 Monaten keine Polymer- Separatlons- und Füllstoff Sedimentationsneigung und keine Nachversteifung auftritt.

3. Abformmaterial nach einem der Ansprüche 1 bis 2 **dadurch gekennzeichnet, dass** durch den Einsatz des ersten Füllstoffs die Reißfestigkeit im ausgehärteten Zustand zwischen 150 bis 250 N/cm², gemessen nach DIN 53504, liegt.

4. Abformmaterial nach Anspruch 1, **dadurch gekennzeichnet, dass** die härtbare Komponente Hydrokolloide, Alginate, Polyether, Kunststoffe, Gipse, kondensationsvernetzende Silikone, additionsvernetzende Silikone, additionsvernetzende Silikonpolyether umfasst.

5. Abformmaterial nach Anspruch 2, **dadurch gekennzeichnet, dass** es additionsvernetzende Silikone und additionsvernetzende Silikonpolyether umfasst.

6. Abformmaterial nach mindestens einem der Ansprüche 1 bis 4, **dadurch gekennzeichnet, dass** es zwischen 20 bis 120 Sekunden verarbeitbar ist und eine Aushärtung bei Mundtemperatur (35°C) innerhalb von 240 Sekunden erfolgt.

7. Abformmaterial nach mindestens einem der Ansprüche 1 bis 6, wobei der erste Füllstoff eine naßgefällte Kieselsäure oder eine natürlich vorkommende Kieselsäure ist, die einen relativ hohen Wassergehalt von 2 - 8 % haben können.

8. Abformmaterial nach mindestens einem der Ansprüche 1 bis 7, wobei der erste Füllstoff eine Dichte von 2,0 bis 2,2 g/cm³, eine Dibutylphalat-Adsorption nach DIN 53601 zwischen 140 und 180 g/100g, eine Öladsorption nach DIN ISO 7875 zwischen 35 bis 60 g/100 und /oder eine mittlere Korngröße zwischen 0,5 und 20 µm aufweist.

9. Abformmaterial nach mindestens einem der Ansprüche 1 bis 8, wobei ein zweiter Füllstoff eine Dichte zwischen 2,0 und 2,2 g/cm³, eine BET-Oberfläche beträgt zwischen 110 und 170 m²/g insbesondere zwischen 130 und 150 m²/g aufweist und/oder die Primärteilchengröße zwischen 5 bis 30 nm liegt.

10. Abformmaterial nach mindestens einem der Ansprüche 1 bis 9, wobei der erste Füllstoff Siliciumdioxid, insbesondere in Mengen von 80 bis 100 %, und Metalloxide wie Aluminiumoxid, Calclumoxid, Natriumoxid, Kaliumoxid, Eisenoxid und Titandioxid, insbesondere in Mengen von 0 bis 20 %, umfasst.

11. Abformmaterial nach mindestens einem der Ansprüche 1 bis 10, wobei der mindestens erste und/oder der mindestens zweite Füllstoff hydrophobisiert ist (sind).

12. Abformmaterial nach mindestens einem der Ansprüche 1 bis 11, wobei der zweite Füllstoff als hydrophobierte Kieselsäure hochdispers vorliegt und durch eine Flammenhydrolyse von Siliciumtetrachlorid hergestellt wurde.

13. Abformmaterial nach mindestens einem der Ansprüche 1 bis 12, **dadurch gekennzeichnet, dass** es sich um additionsvenetzende Silikone mit folgenden Bestandteilen
a) ungesättigte, insbesondere α,ω ungesättigte Polysiloxane, α,ω Alkenylpolysiloxane sowie
b) Organohydrogenpolysiloxane und
c) Katalysatoren handelt.

14. Abformmaterial nach mindestens einem der Ansprüche 1 bis 13, **dadurch gekennzeichnet, dass** als Inhaltsstoffe a) α,ω-Dialkyl-,Diaryl-, Arylalkyl-, α,ω-Alkenyl- und insbesondere Vinylpolysiloxane oder substituierte Dialkyl-, Diaryl-, Arylalkyl-Polysiloxane oder Polyether, insbesondere α, ω - vinylendgestoppte Polydimethylsiloxane in einer Menge von 40 bis 80 Gew. %, insbesondere von 50 bis 70 Gew. %, enthalten sind.

15. Abformmaterial nach mindestens einem der Ansprüche 1 bis 14, **dadurch gekennzeichnet; dass** als Inhaltsstoffe b) Alkyl- oder Aryl-Organohydrogenpolysiloxane, insbesondere Polyhydrogenpolydimethylsiloxane mit mindestens 2 SiH -Gruppen oder einem SiH - Gehalt von 0,1 bis 15 mmol/g in einer Menge von 2 - 40 Gew.%, insbesondere von 10 bis 30 Gew.%, enthalten sind.

16. Abformmaterial nach mindestens einem der Ansprüche 1 bis 15, **dadurch gekennzeichnet, dass** als Inhaltsstoffe c) Katalysatoren der VIII. Nebengruppe, insbesondere Katalysatoren für die Hydrosilyllerung, Salze, komplexe und kolloidal vorliegende Formen der Übergangsmetalle der VIII. Nebengruppe, vorzugsweise die Metalle Platin, Palladium und Rhodium, insbesondere Platinkomplex, die z.B. aus Hexachloroplatinsäure bzw. aus Platinsalzen hergestellt werden, (Karstedt Katalysatoren) in einem Prozentbereich von 0,0001 bis 0,1 Gew. %, insbesondere 0,0005 bis 0,1 Gew. % bezogen auf reines Metall, enthalten sind.

17. Abformmaterial nach mindestens einem der Ansprüche 1 bis 16, wobei der erste Füllstoff in einem Mengenbereich von 10 bis 50 Gew. %, insbesondere 20 bis 30 % Gew. vorliegt.

18. Abformmaterial nach mindestens einem der Ansprüche 1 bis 17, **dadurch gekennzeichnet, dass** es Polysiloxane mit Methacrylat-Gruppen sowie ein Initiatorsystem, insbesondere ein lichtaktivierbares Initiatorsystem, z. B. durch UV-Licht, umfasst.

19. Abformmaterial nach mindestens einem der Ansprüche 1 bis 18, **dadurch gekennzeichnet, dass** die Inhaltsstoffe a) und c) räumlich voneinander getrennt vorliegen.

20. Abformmaterial nach Anspruch 1, **dadurch gekennzeichnet, dass** der erste Füllstoff anorganisch ist und dass ein zweiter Füllstoff enthalten ist, der ein anorganischer verstärkender Füllstoff ist, der eine größere BET-Oberfläche besitzt als die Oberfläche des gewählten ersten Füllstoffes.

21. Abformmaterial nach Anspruch 20, wobei der zweite Füllstoff eine BET-Oberfläche von 50 - 700 m²/g, insbesondere 110 - 170 m²/g aufweist.

22. Ahformmaterial nach Anspruch 20, wobei der erste Füllstoff eine haßgefällte Kieselsäure oder eine natürlich vorkommende Kieselsäure ist, die einen relativ hohen Wassergehalt von 2 - 8 % haben können.

23. Abformmaterial nach Anspruch 20, wobei der erste Füllstoff eine Dichte von 2,0 bis 2,2 g/cm³, eine Dibutylphalat-Adsorption nach DIN 53601 zwischen 140 und 180 g/100g eine Öladsorption nach DIN ISO 7875 zwischen 35 bis 60 g/100 und /oder eine mittlere Korngröße zwischen 0,5 und 20 µm aufweist.

24. Abformmaterial nach mindestens einem der Ansprüche 20 bis 23, wobei der zweite Füllstoff hydrophobiert ist.

## Claims

1. Impression material for use with an impression spoon according to WO-A-97/32536 or WO-A-98/52491 in respect of patients, the impression material comprising hardenable constituents and at least one first filler, **characterized in that** said at least first filler has a BET surface of 20 to 50 m²/g, and preferably 30 to 40 m²/g, and is present in an amount of 20 to 30 % by weight, as a result of which the impression material has a thixotropic index of ≤ 1.1 and attains a viscosity of 1 to 40 Pas, preferably 1-10 Pas.

2. Impression material according to Claim 1, **characterized in that**, due to the use of the first filler, there is no tendency towards polymer separation and filler sedimentation and no subsequent stiffening during the storage period of at least 18 months.

3. Impression material according to one of Claims 1 or 2, **characterized in that**, due to the use of the first filler, resistance to cracking in the hardened state is between 150 and 250 N/cm², measured according to DIN 53504.

4. Impression material according to Claim 1, **characterized in that** the hardenable constituents include hydrocolloids, alginates, polyethers, plastics, gypsums, condensation-cross-linking silicones, addition-cross-linking silicones, addition-cross-linking silicone polyethers.

5. Impression material according to Claim 2, **characterized in that** it includes addition-cross-linking silicones and addition-cross-linking silicone polyethers.

6. Impression material according to at least one of Claims 1 to 4, **characterized in that** it is workable for between 20 and 120 seconds and hardening takes place at mouth temperature (35°C) within 240 seconds.

7. Impression material according to at least one of Claims 1 to 6, the first filler being a wet-precipitated silica or a naturally occurring silica which can have a relatively high water content of 2 - 8 %.

8. Impression material according to at least one of Claims 1 to 7, the first filler having a density of between 2.0 and 2.2 g/cm³, a dibutyl phthalate adsorption according to DIN 53601 of between 140 and 180 g/100g, an oil adsorption according to DIN ISO 7875 of between 35 and 60 g/100 and/or a mean particle size of between 0.5 and 20 µm.

9. Impression material according to at least one of Claims 1 to 8, a second filler having a density of between 2.0 and 2.2 g/cm³, a BET surface amounting to between 110 and 170 m²/g, and in particular between 130 and 150 m²/g, and/or the primary particle size being between 5 and 30 nm.

10. Impression material according to at least one of Claims 1 to 9, the first filler comprising silicon dioxide, in particular in amounts of 80 to 100 %, and metal oxides, such as aluminium oxide, calcium oxide, sodium oxide, potassium oxide, iron oxide and titanium oxide, in particular in amounts from 0 to 20 %.

11. Impression material according to at least one of Claims 1 to 10, the at least first and/or the at least second filler being hydrophobized.

12. Impression material according to at least one of Claims 1 to 11, the second filler being present as hydrophobized silica and having been produced by a flame hydrolysis of silicon tetrachloride.

13. Impression material according to at least one of Claims 1 to 12, **characterized in that** it involves addition-cross-linking silicones with the following constituents:
a) unsaturated, particularly α, ω unsaturated polysiloxanes, α, ω alkenyl polysiloxanes and
b) organohydrogenpolysiloxanes and
c) catalysts.

14. Impression material according to at least one of Claims 1 to 13, **characterized in that** included as components a) are α, ω-dialkyl, diaryl, arylalkyl, α, ω-alkenyl and in particular vinyl polysiloxanes or substituted dialkyl, diaryl, arylalkyl polysiloxanes or polyethers, in particular α, ω-vinyl-terminated polydimethylsiloxanes in an amount of 40 to 80 % by weight, in particular 50 to 70 % by weight.

15. Impression material according to at least one of Claims 1 to 14, **characterized in that** included as components b) are alkyl or aryl organohydrogenpolysiloxanes, in particular polyhydrogenpolydimethylsiloxanes with at least 2 SiH groups or an SiH content of 0.1 to 15 mmol/g in an amount of 2 - 40 % by weight, in particular 10 to 30 % by weight.

16. Impression material according to at least one of Claims 1 to 15, **characterized in that** included as components c) are catalysts of subgroup VIII, in particular hydrosilylation catalysts, salts, complex and colloidal forms of the transition metals of subgroup VIII, preferably the metals platinum, palladium and rhodium, in particular platinum complex, which for example are produced from hexachloroplatinic acid or from platinum salts (Karstedt catalysts) in a percentage range of 0.0001 to 0.1 % by weight, in particular 0.0005 to 0.1 % by weight, based on pure metal.

17. Impression material according to at least one of Claims 1 to 16, the first filler being present in an amount ranging from 10 to 50 % by weight, and in particular 20 to 30 % by weight.

18. Impression material according to at least one of Claims 1 to 17, **characterized in that** it includes polysiloxanes with methacrylate groups as well as an initiator system, in particular an initiator system which can be activated by light, for example UV light.

19. Impression material according to at least one of Claims 1 to 18, **characterized in that** the components a) and c) are present in such a way as to be spatially separate from one another.

20. Impression material according to Claim 1, **characterized in that** the first filler is inorganic and **in that** a second filler is included, which is an inorganic strengthening filler having a larger BET surface than the surface of the selected first filler.

21. Impression material according to Claim 20, the second filler having a BET surface of 50 to 700 m²/g, and in particular 110 - 170 m²/g.

22. Impression material according to Claim 20, the first filler being a wet-precipitated silica or a naturally occurring silica, which can have a relatively high water content of 2 - 8 %.

23. Impression material according to Claim 20, the first filler having a density of 2.0 to 2.2 g/cm³, a dibutyl phthalate adsorption according to DIN 53601 of between 140 and 180 g/100g, an oil adsorption according to DIN ISO 7875 of between 35 and 60 g/100 and/or a mean particle size of between 0.5 and 20 µm.

24. Impression material according to at least one of Claims 20 to 23, the second filler being hydrophobized.

## Revendications

1. Matériau d'empreinte destiné à être utilisé avec une louche d'empreinte selon le document WO-A-97/32536 ou WO-A-98/52491 sur des patients, le matériau d'empreinte contenant des composants durcissables et au moins un agent de remplissage, **caractérisé en ce que** le au moins premier agent de remplissage a une surface BET comprise entre 20 et 50 m²/g, de préférence entre 30 et 40 m²/g, et est présent dans une quantité comprise entre 20 et 30 % en poids, de sorte que le matériau d'empreinte a un index de thixotropie ≤ 1,1 et une viscosité comprise entre 1 et 40 Pas, de préférence entre 1 et 10 Pas.

2. Matériau d'empreinte selon la revendication 1, **caractérisé en ce que** en raison de l'utilisation du premier agent de remplissage, il ne se produit pas de tendance à la polymérisation, à la séparation et à la sédimentation de l'agent de remplissage ni de rigidification postérieure pendant la durée de conservation d'au moins 18 mois.

3. Matériau d'empreinte selon l'une des revendication 1 ou 2, **caractérisé en ce que** en raison de l'utilisation du premier agent de remplissage, la résistance à la rupture à l'état durci est comprise entre 150 et 250 N/cm², mesurée conformément à la norme DIN 53504.

4. Matériau d'empreinte selon la revendication 1, **caractérisé en ce que** les composants durcissables comprennent des hydrocolloïdes, de alginates, des polyéthers, des matières plastiques, du plâtre, des silicones réticulants par condensation, des silicones réticulants par addition, des polyéthers de silicone réticulants par addition.

5. Matériau d'empreinte selon la revendication 2, **caractérisé en ce qu'**il comprend des silicones réticulants par addition et des polyéthers de silicone réticulants par addition.

6. Matériau d'empreinte selon l'une au moins des revendications 1 à 4, **caractérisé en ce qu'**il peut être travaillé entre 20 et 120 secondes et que le durcissement se produit à température buccale (35 °C) en l'espace de 240 secondes.

7. Matériau d'empreinte selon l'une au moins des revendications 1 à 6, le premier agent de remplissage étant un acide silicique précipité par voie humide ou un acide silicique présent à l'état naturel pouvant avoir une relativement importante teneur en eau de 2 à 8 %.

8. Matériau d'empreinte selon l'une au moins des revendications 1 à 7, le premier agent de remplissage ayant une densité comprise entre 2,0 et 2,2 g/cm³, une adsorption du dibutylphtalate selon la norme DIN 53601 comprise entre 140 et 180 g/100g et une adsorption de l'huile selon la norme DIN 7875 comprise entre 35 et 60 g/100 et / ou une granulométrie moyenne comprise entre 0,5 et 20 µm.

9. Matériau d'empreinte selon l'une au moins des revendications 1 à 8, un deuxième agent de remplissage ayant une densité comprise entre 2,0 et 2,2 g/cm³, une surface BET comprise entre 110 et 170 m²/g, notamment comprise entre 130 et 150 m²/g, et / ou une granulométrie primaire comprise entre 5 et 30 nm.

10. Matériau d'empreinte selon l'une au moins des revendications 1 à 9, le premier agent de remplissage comprend du dioxyde de silicium, notamment en une quantité comprise entre 80 et 100 %, et des oxydes métalliques tels que l'oxyde d'aluminium, de l'oxyde calcium, de l'oxyde de sodium, de l'oxyde de potassium, de l'oxyde de fer et de l'oxyde de titane, notamment en une quantité comprise entre 0 et 20 %.

11. Matériau de remplissage selon l'une au moins des revendications 1 à 10, le premier et / ou le second agent(s) de remplissage est/sont rendus hydrophobe(s).

12. Matériau d'empreinte selon l'une au moins des revendications 1 à 11, le second agent de remplissage est présent sous la forme d'acide silicique rendu hydrophobe hautement dispersé et a été obtenu par hydrolyse au chalumeau oxhydrique à partir de tétrachlorure de silicium.

13. Matériau d'empreinte selon l'une au moins des revendications 1 à 12, **caractérisé en ce qu'**il s'agit de silicone réticulant par addition avec les composant suivants :
a) des polysiloxanes insaturés, notamment α,ω insaturés, des α,ω alkenylpolysiloxanes ainsi que
b) des organohydrogénopolysiloxanes et
c) des catalyseurs.

14. Matériau d'empreinte selon l'une au moins des revendications 1 à 13, **caractérisé en ce que** sont compris comme constituants a) des polysiloxanes de α,ω-dialkyle, de diaryle, d'alrylalkyle, de α,ω-alkenyle et notamment de vinyle, ou des polysiloxanes substitués de dialkyle, de diaryle, d'arylalkyle ou des polyéthers, notamment des α,ω polydiméthylsiloxanes arrêtés à l'extrémité vinyle en une quantité comprise entre 40 et 80 % en poids, notamment comprise entre 50 et 70 % en poids.

15. Matériau d'empreinte selon l'une au moins des revendications 1 à 14, **caractérisé en ce que** sont compris comme constituants b) des organohydrogénopolysiloxanes d'alkyle ou d'aryle, notamment des polyhdrogénopolydiméthylsiloxanes avec au moins 2 groupes SiH ou une teneur en SiH comprise entre 0,1 et 15 mmol/g dans une quantité de 2 à 40 % en poids, notamment de 10 à 30 % en poids.

16. Matériau d'empreinte selon l'une au moins des revendications 1 à 15, **caractérisé en ce que** sont compris comme constituants c) des catalyseurs du sous-groupe VIII, notamment des catalyseurs pour l'hydrosilylation, des sels, des formes complexes et colloïdales des métaux de transition du sous-groupe VIII, de préférence les métaux platine, palladium et rhodium, en particulier des complexes de platine qui sont obtenus à partir d'acide hexachloroplatinique ou à partir de sels de platine, (catalyseurs de Karstedt) dans un rapport de pourcentage de 0,0001 à 0,1 % en poids, notamment de 0,0005 à 0,1 % en poids rapporté au métal pur.

17. Matériau d'empreinte selon l'une au moins des revendications 1 à 16, le premier agent de remplissage étant présent en une quantité comprise entre 10 et 50 % en poids, notamment entre 20 et 30 % en poids.

18. Matériau d'empreinte selon l'une au moins des revendications 1 à 17, **caractérisé en ce qu'**il inclut des polysiloxanes avec des groupes méthacrylates ainsi qu'un système amorceur, notamment un système amorceur pouvant être activé par la lumière, par exemple par la lumière UV.

19. Matériau d'empreinte selon l'une au moins de revendications précédentes, **caractérisé en ce que** les constituants a) et les constituants c) sont séparés dans l'espace les uns des autres.

20. Matériau d'empreinte selon la revendication 1, **caractérisé en ce que** le premier agent de remplissage est minéral et **en ce qu'**un second agent de remplissage est présent qui est un agent de remplissage minéral de renfort dont la surface BET est plus grande que la surface du premier agent de remplissage choisi.

21. Matériau d'empreinte selon la revendication 20, le deuxième matériau de remplissage ayant une surface BET de 50 à 700 m²/g, notamment de 110 à 170 m²/g.

22. Matériau d'empreinte selon la revendication 20, le premier agent de remplissage étant un acide silicique précipité par voie humide ou un acide silicique présent à l'état naturel, qui peuvent avoir une relativement importante teneur en eau de 2 à 8 %.

23. Matériau d'empreinte selon la revendication 20, le premier agent de remplissage ayant une densité de 2,0 à 2,2 g/cm³, une adsorption du dibutylphtalate selon la norme DIN 53601 comprise entre 140 et 180 g/100g, une adsorption de l'huile selon la norme DIN ISO 7875 comprise entre 35 et 60 g/100 et / ou une granulométrie moyenne comprise entre 0,5 et 20 µm.

24. Matériau d'empreinte selon l'une au moins des revendications 20 à 23, le deuxième agent de remplissage est rendu hydrophobe.
